# EUROPEAN PATENT APPLICATION

(11) **EP 4 756 429 A2**
(43) Date of publication of application: **10.06.2026**
(21) Application number: 26159866.8
(22) Date of filing: 08.09.2022
(51) Int. Cl.: G01N 33/569

(54) **METHOD AND KIT FOR THE DETECTION OF BOVINE HERPES VIRUS TYPE 1 (BOHV-1) ANTIBODIES**

(30) Priority: 11.09.2021 EP 21196189
(62) Divisional of application: 22194619.7
(71) Applicant: IDEXX Laboratories, Inc., Westbrook, ME 04092 (US)
(72) Inventor: Abicht, Helge Karl Friedrich, Bern (CH); Sénéchal, Yann Julien, Ecuvillens (CH)
(74) Representative: Hoefer & Partner Patentanwälte mbB

(57) **Abstract**

The invention relates to novel methods, kits and use of polypeptides for the detection of antibodies against Bovine Herpes Virus Type 1 (BoHV-1) infection in biological samples including milk, pooled milk and bulk milk.

## Description

The invention relates to novel methods, kits and use of polypeptides for the detection of antibodies against Bovine Herpes Virus Type 1 (BoHV-1) in individual and pooled milk samples or in samples of serum or plasma.

### Background

Bovine Herpes Virus Type 1 (BoHV-1) causes IBR (infectious bovine rhinotracheitis) and is highly contagious, affects respiratory tract and reproductive system. The disease has a significant economic impact because it leads to a reduction in milk yield, weight loss, abortions, infertility and higher susceptibility to secondary bacterial infections. Consequently, several European countries established control and programs which led to the eradication of the disease in some countries (Switzerland, Austria, Denmark, Germany, Finland, Sweden) and reduction of disease prevalence in others (France, Belgium, Czech Republic).

One cornerstone of BoHV-1 control and surveillance programs is the screening of animals for presence of antibodies directed against the virus. For serological testing of individual animals enzyme-linked immunosorbent assays (ELISA) based on detection of antibodies against the whole virus or the two viral glycoproteins gB and gE are used. For testing on a herd level indirect tank milk ELISA tests using whole virus preparations as antibody targets have been proven to be superior in sensitivity to gB or gE blocking ELISA tests, hence indirect ELISA tests are used as the main tool for surveillance testing in disease-free countries like Germany, Austria and Switzerland.

Current test methods suffer from the occurrence of false positive results due to non-specific binding of antibodies to complex antigens like whole virus preparations coated on microtiter plates or interference of antibodies directed against closely related pathogens like BoHV-2. Those so called "singleton reactors" are causing severe issues in control programs because of the economic impact on farms due to trade restrictions. Current countermeasures to avoid non-feasible singleton reactors include use of negative control wells for each sample in indirect ELISA tests (bi-phasic ELISA), re-testing of pooled samples with tests of lower sensitivity but higher specificity (gB and gE blocking ELISA tests) and testing of individual serum samples of each cow contributing to a pooled sample using gB ELISA tests and virus neutralization assays in case of an assumed BoHV-2 involvement. All countermeasures are costly, time consuming and labor-intensive.

Known methods and kits for the detection of antibodies against BoHV-1 imply a number of disadvantages and are not as reliable as needed.

There was a need for a diagnostic test combining high sensitivity and high specificity to eliminate the use of control wells (costs and labor time) and minimize the rate of singleton reactors (trade bans and re-testing of animals).

Hence it was one object underlying the invention to providing an effective method for the detection of antibodies against BoHV-1.

It was another and particular object underlying the invention to providing a method for detection of antibodies against BoHV-1 in bulk milk of pooled or individual samples from cows.

It was another object underlying the invention to providing a method for detection of antibodies against BoHV-1 in samples of serum or plasma.

It was yet another object underlying the invention to providing a kit useful in the detection of antibodies against BoHV-1 and in particular useful in bulk milk of pooled or individual samples from cows, or in samples of serum or plasma.

### Summary of the Disclosure

In one aspect the invention is directed to novel polypeptides useful in the detection of BoHV-1 antibodies.

In another aspect the invention is directed to a method of making a protein or polypeptide useful in the detection of antibodies against BoHV-1.

In another aspect the invention is directed to a method of detection of antibodies against BoHV-1.

In another aspect the invention is directed to the use of novel polypeptides in the detection of antibodies against BoHV-1.

In another aspect the invention is directed to a kit useful in the detection of antibodies against BoHV-1.

In another aspect the invention is directed to a nucleic acid encoding a polypeptide as disclosed herein.

In another aspect the invention is directed to method as disclosed herein further comprising treating the non-human animal with one or more antivirals, one or more non-steroidal anti-inflammatories, and/or one or more antibiotics if a complex is detected in the sample.

### Brief Description of the Figures

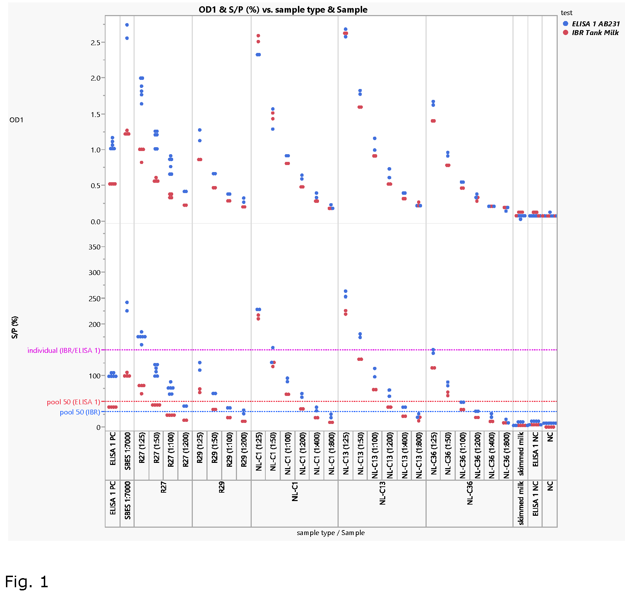

Fig. 1 shows that the analytical sensitivity of the gC-double-antigen-sandwich (DAS)-ELISA is comparable to IDEXX BHV-1 Bulk Milk (ELISA 1) . PC, positive control; NC, negative control; R27/R29, German reference samples; SBES, a BoHV-1 antibody positive serum used as positive control for the gC-DAS-ELISA; NL-C1/NL-C13/NL-C36, milk field samples of cows infected with BoHV-1 from the Netherlands;
   Detectability thresholds of the gC-based assay were comparable to classical tests using whole virus-derived antigens.

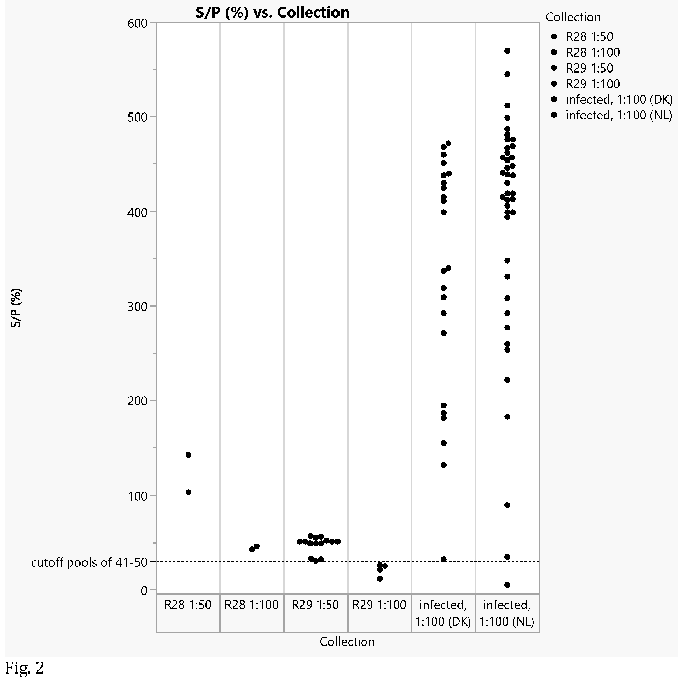

Fig. 2 shows that the gC-DAS-ELISA is highly sensitive in pools of 100 milk samples. R28 1:50, R28 1:100, R29 1:50, and R29 1:100 : German reference samples diluted 1:50 or 1:100, respectively; infected 1:100 (DK) and infected 1:100 (NL) : Milk field samples of cows infected with BoHV-1 from Denmark (n=23) and the Netherlands (n=41), respectively.
   In sum, diagnostic and analytical sensitivity data of the tests and kits according to the disclosure show that gC can be used as a single recombinant antigen instead of whole virus-based antigens without any gap in detection due to "non gC reactors" or weak reactivity.

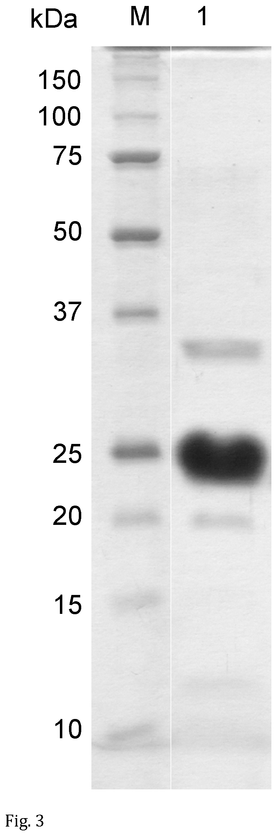

Fig. 3 depicts a SDS PAGE of gC-Nterm. *M,* molecular weight marker (MW of the proteins present in the marker is indicated on the left side of the figure); *Lane 1,* 1.3 ug gC-Nterm.

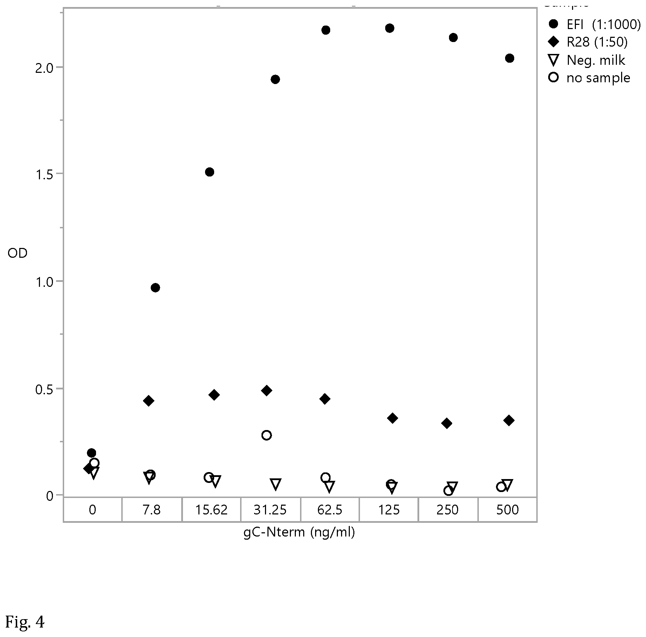

Fig. 4 depicts the N-terminus of BoHV-1 gC can be used as antigen in an indirect ELISA. Samples were prediluted in commercial skimmed milk and tested in an indirect ELISA using plates coated with gC-Nterm at the concentration indicated on the x-axis. *Closed symbols,* positive samples; *open symbols,* negative samples; *EFI (1:1000),* BoHV-1 Ab positive serum diluted 1000-fold in commercial skimmed milk; *R28 (1:50),* German BoHV-1 Ab positive reference milk sample diluted 1:50 in commercial skimmed milk; *Neg. milk,* BoHV-1 Ab negative commercial skimmed milk.

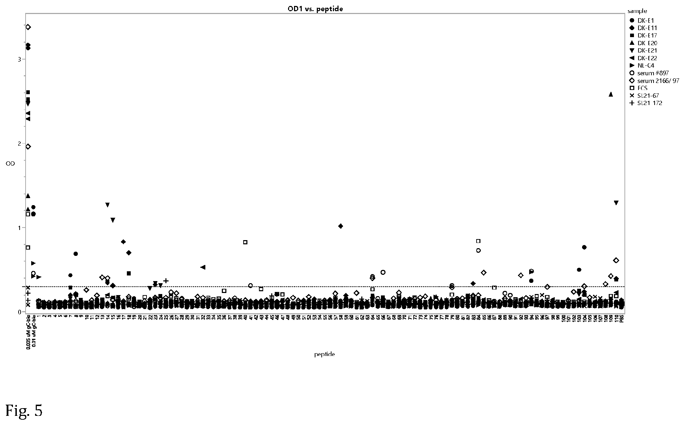

Fig. 5 depicts the BoHV-1 gC epitope mapping - screening ELISA using a gC peptide array and bovine samples. *Filled symbols,* IgG from BoHV-1 Ab positive milk; *open symbols,* IgG from BoHV-1 Ab positive serum; *x and +,* IgG from BoHV-1 Ab negative milk. *Dashed line:* cutoff; *FCS,* fetal calf serum (i.e. FBS); *gC-bio,* biotinylated soluble domain of gC (Ala26-Ser474).

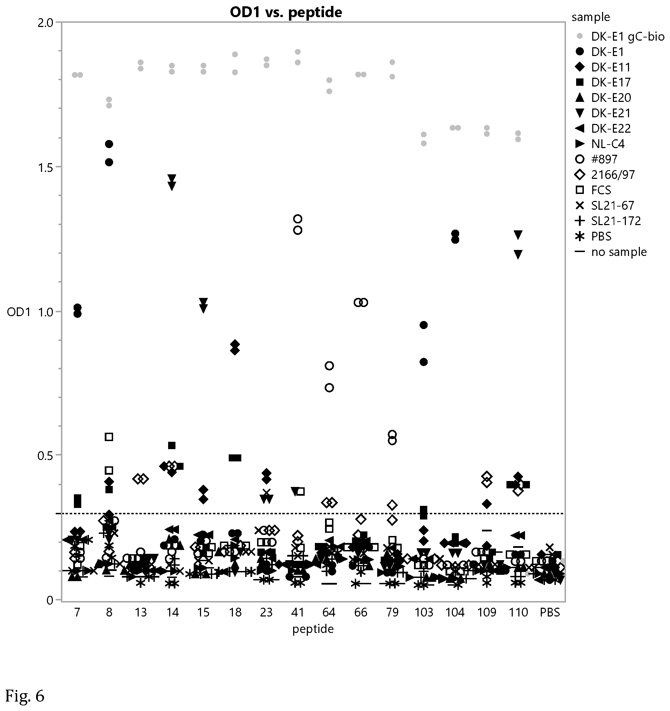

Fig.6 depicts the BoHV-1 gC epitope mapping - confirmatory ELISA. Samples reacting with a peptide in the screening ELISA were retested in duplicate with each peptide identified during screening. *Filled symbols,* IgG from BoHV-1 Ab positive milk; *open symbols,* IgG from BoHV-1 Ab positive serum; *x and +,* IgG from BoHV-1 Ab negative milk; *dashed line:* cutoff; *PBS,* phosphate buffered saline; *DK-E1 gC-bio,* ELISA positive control i.e. BoHV-1 Ab positive sample DK-E1 was reacted with immobilized gC-biotin and this condition was run in parallel to each peptide ELISA.

### Detailed Description

The disclosure of the invention is described in the following in more detail. The following definitions of important terms of the invention will apply throughout the specification, figures, examples and claims. Any additional terms will be understood by the skilled person according to the general knowledge in the field.

"BoHV-1" as used herein refers to the Bovine Herpes Virus 1. BoHV-1 is a member of the genus Varicellovirus, subfamily *Alphaherpesvirinae,* and causes reproductive, respiratory, enteric, and neurologic disease in cattle. The infection is characterized by upper respiratory tract disease with a purulent nasal discharge, hyperemia of the muzzle and conjunctivitis. The BoHV-1 is composed of about 33 proteins. One is the glycoprotein C (gC) which serves for cell attachment. The N-terminal or soluble domain of said polypeptide can be produced with known biotechnology procedures, e.g. it can be produced in E. coli or HEK cells or CHO or Expi293 cells, known to the skilled person.

The BoHV-1 gC protein or a fragment or a polypeptide thereof can serve as antigen (Ag) for coating a solid support, like a multiwell plate or a test strip, and the same or a different gC or fragment thereof or a polypeptide thereof can serve as means for visualization by way of coupling a marker to said gC antigen.

"Amino acid" refers to naturally occurring and synthetic amino acids. Amino acid residues are abbreviated as follows: Alanine is A or Ala; Arginine is R or Arg; Asparagine is Nor Asn; Aspartic Acid is D or Asp; Cysteine is C or Cys; Glutamic Acid is E or Glu; Glutamine is Q or Gln; Glycine is G or Gly; Histidine is H or His; Isoleucine is I or Ile; Leucine is L or Leu; Lysine is K or Lys; Methionine is M or Met; Phenylalanine is F or Phe; Proline is P or Pro; Serine is S or Ser; Threonine is T or Thr; Tryptophan is W or Trp; Tyrosine is Y or Tyr; and Valine is V or Val. Except where defined otherwise herein, X or Xaa represents any amino acid. Other relevant amino acids include, but are not limited to being, 4-hydroxyproline and 5-hydroxylysine. In all cases, the amino acid sequence of a polypeptide described or otherwise referred to herein is presented in conventional form in that the left-most, or first, amino acid residue of the sequence is the N- terminal residue and the right-most, or last, amino acid residue of the sequence is the C-terminal residue.

A "conservative variant" of any particular nucleic acid sequence includes any sequence having one or more degenerate codon substitutions to that particular nucleic acid sequence, any sequence having one or more nucleotide substitutions to, insertions to, and deletions from that particular nucleic acid sequence, and the complementary sequence of that particular nucleic acid and the conservative variants of that complementary sequence. Conservative variants of a particular nucleic acid sequence preferably have at least about 78% identity, more preferably have at least about 90% identity, and even more preferably at least about 95-99% identity, to that particular nucleic acid sequence. Conservative variants of a particular nucleic acid sequence may be artificially synthesized or they may be isolated in their natural form from an organism.

A "conservative variant" of any particular polypeptide sequence is any polypeptide having an amino acid sequence that varies from the amino acid sequence of that particular polypeptide but still retains the specific binding properties of that particular polypeptide, such that an antibody of the present invention that is raised against the particular polypeptide is capable of specifically binding the variant polypeptide. Therefore, for example, a conservative variant of a particular polypeptide may have one or more amino acid substitutions, deletions, additions, and insertions to that particular polypeptide. For example, a conserved variant of a particular polypeptide may have 30 or fewer, 25 or fewer, 20 or fewer, 15 or fewer, 10 or fewer, or 5 or fewer, conserved amino acid substitutions to that particular polypeptide. Conservative variants of a particular polypeptide preferably, but not essentially, have at least about 78% identity, more preferably have at least about 90% identity, and even more preferably at least about 91-99% identity, to that particular polypeptide. A percent identity for any subject nucleic acid or amino acid sequence (e.g., any of polypeptides described herein) relative to another "target" nucleic acid or amino acid sequence can be determined as follows. First, a target nucleic acid or amino acid sequence of the invention can be compared and aligned to a subject nucleic acid or amino acid sequence, using the BLAST 2 Sequences (BI2seq) program from the stand-alone version of BLASTZ containing BLASTN and BLASTP (e.g., version 2.0.14). The stand-alone version of BLASTZ can be obtained at www.ncbi.nlm.nih.gov. Instructions explaining how to use BLASTZ, and specifically the BI2seq program, can be found in the 'readme' file accompanying BLASTZ. The programs are also described in detail by Karlin et al. (1990) Proc. Natl. Acad. Sci. 87:2264;Karlin et al. (1990) Proc. Natl. Acad. Sci. 90:5873;and Altschul et al. (1997) Nucl. Acids Res. 25:3389.

As used herein, the term "peptide" or "polypeptide" refers to a compound of a single chain of amino acid residues linked by peptide bonds. The chain may be of any length. The polypeptide is capable of binding one or more antibodies specific to one or more epitopes of the BoHV-1 polypeptides and preferably to recognize the BoHV-1. The sequence or/and epitope can consist e.g. of between 3 and 25 amino acids, preferably of 5 to 12 amino acids. The polypeptides of the present **disclosure** are capable of eliciting an immune response in a host animal and/or are capable of binding an antibody present in a sample of an infected animal due to an immune response after infection.

An "epitope" or "antigenic determinant" is the sequence stretch in two or three dimensional configuration on a polypeptide of BoHV-1 that is recognized for binding by a paratope on antibodies specific to said BoHV-1 polypeptide. Antibodies in the context of the invention recognize particular epitopes having a sequence of 3 to 15, preferably 5 to 10, more preferable 5 to 7 amino acids. The antibody may further be characterized by its binding affinity to the protein, polypeptide or peptide applied in the methods and kits of the invention and the binding affinity is in the range of 10 ⁻⁹ to 10 ⁻¹⁰ nM. Particular antibodies recognized in the invention are monoclonal or polyclonal antibodies, or fragments thereof directed against a gC polypeptide according to the disclosure and applied as detection means.

The term "label," as used herein, refers to a detectable compound, composition, or solid support, which can be conjugated directly or indirectly (e.g., via covalent or non-covalent means, alone or encapsulated) to a polypeptide or polyclonal or monoclonal antibody or fragments thereof. The label may be detectable by itself (e.g., radioisotope labels, chemiluminescent dye, electrochemical labels, metal chelates, latex particles, or fluorescent labels) or, in the case of an enzymatic label, may catalyze chemical alteration of a substrate compound or composition which is detectable (e.g., enzymes such as horseradish peroxidase, alkaline phosphatase, and the like). The label employed in the current invention could be, but is not limited to alkaline phosphatase; glucose-6-phosphate dehydrogenase ("G6PDH"); horseradish peroxidase (HRP); chemiluminescers such as isoluminol, fluorescers such as fluorescein and rhodamine compounds; ribozymes; and dyes. The label may also be a specific binding molecule which itself may be detectable (e.g., biotin, avidin, streptavidin, digoxigenin, maltose, oligohistidine, 2, 4-dinitrobenzene, phenylarsenate, ssDNA, dsDNA, and the like). The utilization of a label produces a signal that may be detected by means such as detection of electromagnetic radiation or direct visualization, and that can optionally be measured.

A gC polypeptide of the disclosure can be linked to a label using methods well known to those skilled in the art. (E.g., Immunochemical Protocols; Methods in Molecular Biology, Vol. 295, edited by R. Bums (2005)). The detectable gC polypeptide conjugate may be used in any known diagnostic test format, e.g. ELISA format, to generate a signal that is related to the presence or amount of specific antibodies against BoHV-1 in a test sample.

"Substantial binding" or "substantially bind" refer to an amount of specific binding or recognizing between molecules in an assay mixture under particular assay conditions. In its broadest aspect, substantial binding relates to the difference between a first molecule's incapability of binding or recognizing a second molecule, and the first molecules capability of binding or recognizing a third molecule, such that the difference is sufficient to allow a meaningful assay to be conducted to distinguish specific binding under a particular set of assay conditions, which includes the relative concentrations of the molecules, and the time and temperature of an incubation. In another aspect, one molecule is substantially incapable of binding or recognizing another molecule in a cross-reactivity sense where the first molecule exhibits a reactivity for a second molecule that is less than 25%, preferably less than 10%, more preferably less than 5%, most preferably less than 1% of the reactivity exhibited toward a third molecule under a particular set of assay conditions, which includes the relative concentration and incubation of the molecules. Specific binding can be tested using a number of widely known methods, e.g., an immunohistochemical assay, an enzyme-linked immunosorbent assay (ELISA), and any ELISA format described herein, a radioimmunoassay (RIA), surface plasmon resonance, or a western blot assay.

A "biological sample" refers to a sample from an animal subject (or a pool of samples from one or more animal subjects) including saliva, whole blood, serum, plasma, milk, whole milk, skim milk, bulk milk, tank milk or other sample that may contain anti-BoHV-1 antibodies. A biological sample can be collected and stored by any convenient means that does not impact the ability of the sample to be tested in the method of the invention. Samples for use in the method of the invention may require preparation prior to use in the method. For example, samples may need to be precipitated, diluted, filtered or centrifuged to remove unwanted components or components may be added to the sample such as salts, detergents or additional proteins.

The term "solid support" or "solid phase" refers to a non-aqueous matrix to which a BoHV-1 polypeptide or an antibody against BoHV-1 can adhere by covalent or non-covalent binding. Examples of solid support include supports formed partially or entirely of glass (e.g., controlled pore glass), synthetic and natural polymers, polysaccharides (e.g., agarose), polyacrylamides, polystyrene, polyvinyl alcohols and silicones, magnetic or paramagnetic particles, barcoded particles, latex particles, chromatographic strips, microtiter polystyrene plates, or any other substances that will allow bound antigens to be washed or separated from unbound materials. In certain embodiments, depending on the application, the solid support can be the well of an assay plate or can be a purification column (e.g., an affinity chromatography column).

As used herein, the term "kit" or "assay kits" (e.g., articles of manufacture) refers to an assembly of useful compounds and other means like solid support plates or test stripes for detecting antibodies against BoHV-1 in a mammalian sample, preferably ruminants. A kit therefore may include one or more devices and/or compositions of the present invention. In one particular example, such a kit includes the device having an immobilized BoHV-1 polypeptide, fragment thereof, and comprising one or more labeled BoHV-1 polypeptide and other useful reagents like wash reagent, as well as detector reagent and positive and negative control reagents, if desired or appropriate. Other components such as buffers, controls, and the like, known to those of ordinary skill in art, may be included in such test kits. The relative amounts of the various reagents can be varied, to provide for concentrations in solution of the reagents that substantially optimize the sensitivity and specificity of the assay. Particularly, the reagents can be provided as dry powders, usually lyophilized, which on dissolution will provide for a reagent solution having the appropriate concentrations for combining with a sample. The present kit may further include instructions for carrying out one or more methods of the present invention, including instructions for using any device and/or composition of the present invention that is included with the kit.

The problem underlying the application was solved by an isolated polypeptide sequence of BoHV-1 gC comprising or consisting of SEQ ID NO. 3 or 4, or any of SEQ ID Nos. 6 to 128 or a combination of any thereof or a fragment thereof, preferably having at least 90% sequence identity with of SEQ ID NO. 3 or 4, or any of SEQ ID Nos. 6 to 128, preferably at least 95%, more preferably at least 98% sequence identity, or consisting of at least 7, preferably at least 11, more preferably at least 15 contiguous amino acids of an amino acid sequence set forth as SEQ ID NO. 3 or 4, or any of SEQ ID Nos. 6 to 128.

Moreover the problem underlying the application was solved by a method of identifying in a sample a non-human animal infected with BoHV-1 comprising the steps:
i. contacting a sample with a polypeptide as described herein under conditions that complexes of antibodies specific against BoHV-1 in the sample and the polypeptide can form;
ii. detecting the complex;
wherein the detection of the complex is indicative of an infection with BoHV-1.

Moreover the problem underlying the application was solved by a kit for the detection of a BoHV-1 infection in a sample comprising:
i. a polypeptides as described herein; and
ii. a means for detection of a complex formed between the polypeptide and an antibody specific against BoHV-1.

Moreover the problem underlying the application was solved by a method for making or/and isolating a polypeptide as described herein, comprising the steps of i. expressing the polypeptide in a prokaryotic or a eukaryotic cell, preferably Expi293 cells, ii. desalting, iii. one or more ion-exchange, and iv. one or more size exclusion steps.

As one solution according to the disclosure an ELISA test was developed based on the recombinantly expressed viral glycoprotein C (gC) which is different from the target proteins used so far.

In a preferred embodiment, an ELISA format changed from a bi-phasic indirect ELISA to a mono-phasic double antigen sandwich ELISA showed advantageous performance. See e.g. Böttcher et al. (Vet. Microbiol. 2012); Valas et al. (Prev. Vet. Medicine 2019); Isa et al. (Berl Munch 381 Tierarztl Wochenschr 116, 192-196); Tignon et al. (J Virol Meth 2017).

Moreover the problem underlying the application was solved by a method as described above further comprising treating the non-human animal with one or more antivirals, one or more non-steroidal anti-inflammatories, and/or one or more antibiotics if a complex is detected in the sample.

Moreover the problem underlying the application was solved by a nucleic acid sequence encoding a polypeptide as set forth in any of SEQ ID NOs. 3 or 4, or 6 to 128.

Moreover the problem underlying the application was solved by a nucleic acid sequence encoding a polypeptide as set forth in any of claims 1 or 2.

The different aspects of the disclosure imply particular advantages, e.g. the majority of currently commercially available ELISA tests used for IBR screening in milk are biphasic. The reason for this is the need for a so-called control well in addition to the specific antigen well to remove the background due to the non-specific signal occurring with some milk samples. In other words, each sample needs to be tested on the specific antigen well and on the control well to generate a test result. This prior art approach is disadvantageous because it complicates the test and renders such test more time consuming and expensive.

The test and test format according to the disclosure is preferably a gC-based assay which is the first monophasic ELISA test for detecting anti-BoHV-1 antibodies in bovine milk. The test according to the disclosure is also applicable in serum or plasma. The prior art tank milk assays used for IBR screening are using whole virus preparations that are more prone to non-specific binding and less defined than recombinants. Other recombinant protein-based ELISAs according to the prior art are usually suffering from poor sensitivity for pooled tank milk testing.

The inventors achieved positive and superior test results with the test format according to the disclosure as well as a simplified test setup is provided wherein a number of gC polypeptides were selected as useful and effective tools in anti-BoHV-1 antibody detection in bulk milk, or plasma and serum, and BoHV-1 infection.

A double antigen sandwich (DAS) ELISA format according to one aspect of the disclosure could be established using purified gC on the solid phase and the same or a different gC as conjugate detector. This assay format according to one aspect of the invention is showing advantageous differentiation between positive and negative samples.

In another aspect of the disclosure the test format can be a classic indirect ELISA using, e.g. an anti-bovine IgG HRP conjugate. In preferred embodiments blocking and competitive ELISA formats can be applied using purified gC according to the disclosure.

It could be shown that surprisingly the use of a BoHV-1 sequence set forth as SEQ ID NO. 3 or 4 or a fragment thereof, or any of SEQ ID Nos. 6 to 128 or a combination of any thereof, preferably having at least 90% sequence identity with of SEQ ID NO. 3 or 4, or any of SEQ ID Nos. 6 to 128, preferably at least 95%, more preferably at least 98% sequence identity, or consisting of at least 7, preferably at least 11, more preferably at least 15 contiguous amino acids of an amino acid sequence set forth as SEQ ID NO. 3 or 4, or any of SEQ ID Nos. 6 to 128 or a combination of any thereof could show advantageous test results, preferably in milk samples and also surprisingly good results could be achieved when combined in a DAS assay format for the detection of BoHV-1 in a milk sample, pooled milk sample, serum or plasma.

In another aspect of the disclosure an advantageous specificity in the detection assay and method can be achieved by using a double antigen sandwich (DAS) ELISA with a gC-HRP conjugate. The gC part can preferably be a fragment of the full length gC part, or the extracellular domain or a fragment thereof, e.g. a stretch of 40, 35, 30 25, 20, 15 or 10 amino acids. Various HRP conjugation methods can be used according to the disclosure such as direct HRP-conjugation, and complexing of biotinylated-gC with various streptavidin-(poly)-HRP versions.

The resulting assay combining the gC according to SEQ ID. NO. 3 or 4 and fragments thereof or any of SEQ ID Nos. 6 to 128 or a combination of any thereof as described above in combination with a DAS ELISA test format demonstrated superior specificity as compared to the state-of-the-art indirect ELISA assay using anti-bovine IgG as conjugate.

In preferred embodiments the disclosure relates to an isolated polypeptide as described herein selected from the group consisting of the sequences SEQ ID Nos. 3 or 4, or any of SEQ ID Nos. 6 to 128 or a combination of any thereof. Such an isolated polypeptide as described herein can preferably be derived from BoHV-1.

The sequences of preferred polypeptides useful in the methods as described herein are set forth in Table 1, 3, 4 and 5 of Example 3 and relate to any of SEQ ID NOs. 6 to 115, or/and to SEQ ID NOs. 116 to 120, or/and to SEQ ID NOs. 121 to 128.

Polypeptides useful in the methods as described herein can comprise less than a certain number of contiguous naturally occurring BoHV-1 amino acids (e.g., about less than 400, 375, 350, 325, 300, 275, 250, 225, 200, 175, 150, 125, 100, 75, 50, 45, 40, 35, 30, 25, 20, 19, 18, 17, 16, 15, 14, 13, 12, 11, 10, or 9 amino acids (or any range between about 400 and about 8 amino acids)) of SEQ ID NOs:1-4. The fact that these polypeptides are smaller than a full length BoHV-1 polypeptide, or smaller than a soluble domain of a BoHV-1 polypeptide is important because smaller polypeptides can have greater specificity and/or sensitivity than full length polypeptides in BoHV-1 assays. Additionally, these smaller polypeptides can be less expensive to manufacture, and may be obtained at greater purity, than the full length polypeptide.

Furthermore, an isolated polypeptide as described herein can be produced in prokaryotic or eukaryotic cells, preferably in CHO or Expi293 or HEK cells. In addition, an isolated polypeptide as described herein can be produced synthetically.

An isolated polypeptide as described herein can preferably comprise an antigenic determinant that is recognized by antibodies specific for BoHV-1.

An isolated polypeptide as described herein can preferably be characterized in that the one or more isolated polypeptides are linked to an indicator reagent, a signal sequence, a stop transfer sequence, an amino acid spacer, a transmembrane domain, a protein purification ligand, a heterologous polypeptide, a moiety that enhances an immune response, a moiety that facilitates purification, a moiety that facilitates polypeptide stability, one or more additional polypeptides as described herein or a combination thereof.

In further preferred embodiments the disclosure relates to a method as described herein wherein the complex is detected by labeling said complex with a detectable agent. The detectable agent can preferably be conjugated to a polypeptide as described herein.

The method as described herein can be characterized that the detectable agent is selected from biotin*SA-HRP, an enzyme, a fluorophore, and a colorimetric agent.

Biotinylation is a technique known to the skilled person and the biotin and biotinylation process can be selected form e.g. enzymatic, primary amine, sulfhydryl, carboxyl, glycoprotein, oligonucleotide or non-specific biotinylation processes and can be chosen from single or poly biotinylation. See e.g. R.A. Kohanski, in Encyclopedia of Biological Chemistry (Second Edition), 2013.

In a preferred method as described herein the polypeptide can be immobilized on a solid support. In yet another preferred embodiment an antibody specific against BoHV-1 is immobilized on a solid support.

Any useful detection method can be applied in combination with the isolated polypeptide as disclosed herein. In a preferred method as described herein the method is an ELISA (enzyme linked immune-sorbent assay), preferably a multi-step or single-step method. In other preferred embodiments the ELISA format can be selected from an indirect, competitive, or blocking ELISA, or combinations thereof.

The method as described herein can be applied with any suitable sample and any sample usually applied in such detection methods. The method as described herein preferably also can be applied and shows advantageous results wherein the sample is whole milk or tank milk. The method can use in preferred embodiments samples from serum or plasma.

In a preferred embodiment of the disclosure the test is a monophasic test which does not need a data/results subtraction step with a control, e.g. a control well. It can be advantageous that also an elution step is not needed in a preferred embodiment according to the disclosure simplifying the format even further. Thus the method and/or kit in a preferred embodiment of the disclosure are directed to a monophasic test format without elution step.

The method and kit according to the disclosure exhibits a high specificity and sensitivity. Preferably the specificity is 94% or higher, 97% or higher, 98% or higher, or 98.5% or higher or substantially 100%.

It is preferred that the sensitivity is at least 95%, more preferably at least 98% and more preferably at least 99%, e.g. 99,3%, or substantially 100%.

In a preferred embodiment the antigen is a recombinant BoHV-1 gC, with a biotinylation tag and a 6His-tag and the conjugate is a gC-biotin*SA-HRP complex wherein the gC is selected from a polypeptide or fragment of SEQ ID NO. 3 or 4, or a polypeptide having at least 95%, more preferably at least 98% sequence identity, or consisting of at least 10 or 15 contiguous amino acids of an amino acid sequence set forth as SEQ ID NOs. 3 or 4.

One example is shown in the sequence of an expression vector as disclosed herein.

Particularly advantageous epitopes have been identified and are depicted in the following Table:

| **Peptide no.** | **Sequence** | **Length** |
|---|---|---|
| 7 | TTGATPPTPNSPDAT | 15 |
| 8 | TPPTPNSPDATPEDS | 15 |
| 13 | TPVGTPEPPSVSEHD | 15 |
| 14 | TPEPPSVSEHDPPVT | 15 |
| 15 | PSVSEHDPPVTNSTP | 15 |
| 18 | STPPPAPPEDGRPGG | 15 |
| 23 | RDGRPSGGGRPRPPR | 15 |
| 41 | GDDEAVRNPFPRAPP | 15 |
| 64 | QPALEGAGYAAVCRA | 15 |
| 66 | YAAVCRAAEYYPPRS | 15 |
| 79 | EDATPTAHPPNLRCD | 15 |
| 103 | RLLSTTDGPVDYTCT | 15 |
| 104 | TTDGPVDYTCTATGY | 15 |
| 109 | EFSATATYDASPGLI | 15 |
| 110 | TATYDASPGLIGS | 13 |

One or more of the above peptides can be applied in the disclosure and in particular the methods as above described. One or more of these peptides show advantageous results in the methods as described and claimed herein.

In some embodiments, the methods described herein can further include administering to the animal one or more treatments comprising non-steroidal anti-inflammatories, antibiotics to manage secondary bacterial infections, antivirals, reduction of stress, isolation, and/or provision of adequate food and water.

Certain embodiments provide methods for diagnosing and treating a disease condition in a subject. The methods comprise a test to determine the presence of anti-BoHV-1 antibodies in a sample from the subject. Treatment is administered to the subject for the disease condition if the sample contains anti-BoHV-1 antibodies as described herein.

Treatment of BoHV-1 infection in infected individuals may include non steroidal anti-inflammatories, antibiotics to manage secondary bacterial infections, antivirals, reduction of stress, isolation from the rest of the herd, and/or provision of adequate food and water.

Treatment with antibiotics may include danofloxacin, ampicillin, enrofloxacin, ceftiofur, tilmicosin, florfenicol, penicillin, tetracycline, oxytetracycline, gamithromycin, and/or tildipirosin. Treatment with non steroidal anti-inflammatories may include flunixin, meloxicam, ketoprofen, and/or firocoxib.

### Examples

This disclosure of the invention is further illustrated by the following examples, which should not be construed as limiting.

### Example 1

### Materials and methods

### Construction of a plasmid for expression of recombinant glycoprotein C of bovine herpesvirus I (pHA25401)

DNA encoding amino acids Ala26-Ser474 of glycoprotein C (gC) of bovine herpesvirus I (BoHV-1) (accession NP_045314.1), a biotinylation tag and a 6His-tag was synthesized together with a 5 '-AscI- and a 3 '-NotI-restriction site, and cloned into pSecTag2-hygroA (Thermo Fisher Scientific, Basel, Switzerland), via the AscI- and NotI-restriction sites, yielding pHA25401. The resulting open reading frame translates into the following amino acid sequence (the native sequence of glycoprotein C (i.e., SEQ ID NO:3) is underlined):

METDTLLLWVLLLWVPGSTGDAAQPARRAEEAEASPSPPPSPSPTETESSAGTTGA
TPPTPNSPDATPEDSTPGATTPVGTPEPPSVSEHDPPVTNSTPPPAPPEDGRPGGA
GNASRDGRPSGGGRPRPPRPSKAPPKERKWMLCEREAVAASYAEPLYVHCGVAD
NATGGARLELWFQRVGRFRSTRGDDEAVRNPFPRAPPVLLFVAQNGSIAYRSAEL
GDNYIFPSPADPRNLPLTVRSLTAATEGVYTWRRDMGTKSQRKVVTVTTHRAPAV
SVEPQPALEGAGYAAVCRAAEYYPPRSTRLHWFRNGYPVEARHARDVFTVDDSGL
FSRTSVLTLEDATPTAHPPNLRCDVSWFQSANMERRFYAAGTPAVYRPPELRVYFE
GGEAVCEARCVPEGRVSLRWTVRDGIAPSRTEQTGVCAERPGLVNLRGVRLLSTT
DGPVDYTCTATGYPAPLPEFSATATYDASPGLIGSGGSGGSSGLNDIFEAQKIEWH
EGSHHHHHH (SEQ ID NO:5)

Amino acids Met1-Gly20 comprise a signal sequence followed by seven non-native amino acids (Asp21-Arg28) derived from the AscI cloning site. The Avi- and the His-tag are separated from the native gC sequence by a GGSGGSS-linker and from each other by a GS-linker. pHA25401 was propagated in *E. coli* Dh5alpha.

### Transient expression of gC in Expi293 cells

Expi293 cells (Thermo Fisher Scientific, Basel, Switzerland) were propagated in Expi293 growth medium i.e. Expi293 expression medium (Thermo Fisher Scientific, Basel, Switzerland) containing 100 U/ml K-penicillin, 100 ug/ml streptomycin-sulfate, 0,05% Pluronic (Gibco, Thermo Fisher Scientific, Basel, Switzerland) and 0,02% AntiClump (Gibco, Thermo Fisher Scientific, Basel, Switzerland) at 37 °C, 8% CO₂, at 120 rpm in single use Erlenmeyer flasks. One day before transfection cells were diluted to a cell density of 2 x 10^6 cells/ml. Before transfection, cell density was adjusted to 3 x 10^6 cells/ml in Expi293 transfection medium (i.e. Expi293 growth medium w/o AntiClump). Transfection was carried out according to manufacturer's instructions using 3.2 ul Expifectamine (Thermo Fisher Scientific, Basel, Switzerland)/ug pHA25401. Plasmid DNA and Expifectamine were premixed in Opti-MEM medium (Thermo Fisher Scientific, Basel, Switzerland) according to manufacturer's instructions. Enhancers were added as indicated by the manufacturer on day 1 after transfection. On day 2 after transfection, 3 g/l glucose were added to the expression culture and cells were incubated for another 4 days. Cells were pelleted at 4800 g for 20 min at RT and the supernatant was either processed the same day or stored at -20 °C for up to one year.

### Purification of gC from cell culture supernatant

To the supernatant 1/9 vol. of 200 mM Tris-Cl, 3 M NaCl, pH 8 were added. To this, 1/24 vol. of 5 M NaCl were added. The resulting solution was filtered through 0.45 um and 0.2 um polyether sulfone filters. The filtered solution was affinity purified on a nickel column. The eluate from the nickel column was pooled and desalted by Sepharose^{®} chromatography. The desalted pool was subjected to anion exchange chromatography on a quaternary ammonium column. The gC protein eluted at approximately 300 mM NaCl. Fractions containing gC were pooled and subjected to size exclusion chromatography (SEC) using a column equilibrated with 20 mM Tris-Cl, 150 mM NaCl, pH 7.4. Fractions containing gC were pooled and 10% glycerol was added as cryoprotectant. The concentration of gC was determined by UV/VIS-spectroscopy. The pooled gC protein was aliquoted, snap-frozen in liquid nitrogen and stored at -80 °C.

### Biotinylation of gC, gC-biotin*Streptavidin-HRP preparation and formulation of the conjugate solution

Biotin was added enzymatically to the polypeptide chain of choice. In general gC-biotin and Streptavidin-HRP were mixed in a predefined molar ratio. The gC-biotin : Streptavidin-HRP complex was then diluted to the desired concentration in a stabilizing agent wherein stabilizing agents known to the skilled person can be applied.

### Coating of 96-well microtiter plates

Purified recombinant BoHV-1 gC glycoprotein was diluted in 0.1 M Na-carbonate, pH 9.5 and 200 ul of this were added to the wells of a 96-well microtiter plates. The plates were incubated for 1 h at RT, washed twice with 300 ul/well PBS, 0.1% Tween 20, and 250 ul of a buffered stabilizing solution containing sugar and a detergent were added to each well, followed by incubation for 1 h at RT. The solution was aspirated and plates were dried.

### Samples

Negative bovine milk samples were collected from IBR-free countries including Germany and Switzerland. BoHV-1 antibody positive milk samples were collected from various vaccinated and/or infected herds originating from Dutch and Danish farms.

German Reference samples were obtained from Friedrich Loeffler Institute (FLI, Germany) and consisted of positive serum (R26 and R27) or milk (R28 and R29) diluted in negative milk. Dilution in negative milk simulates and is equivalent to pooling of the milk of an infected (positive) cow with milks from negative cows. For example, a 1:50 diution of the milk of an infected (positive) cow in negative milk simulates the pooling of the milk of a single infected (positive) cow with milks from 49 negative cows. Such pooling occurs when milks from multiple cows are pooled (combined) in a tank.

### Detailed ELISA test procedure

Milk samples were always skimmed before use (obtained after 15 min. centrifugation at 2000 x g or overnight storage at 2-8°C).

For each ELISA incubation step the plates were tightly sealed or incubated in a humid chamber. A Control Diluent containing salts, a nonionic detergent and a preservative was used as Negative Control (NC). The Positive Control consisted of a BoHV-1 antibody positive serum diluted in Control Diluent. 100 ul sample diluent (containing a detergent and a preservative) were added to the wells and 100 ul of the controls and undiluted skimmed milk samples were dispensed into the wells. The content of the microwells was subsequently mixed by gently tapping the plate or using a microplate shaker. Covered plates were then incubated for 14-18 hours at 2-8°C in a humid chamber. Content of the wells was removed, and each well was washed five times with approximately 300 µL of Wash Solution (10 mM PBS, 0.1% Tween20). Each plate was tapped onto absorbent material after the final wash to remove any residual wash fluid. The Conjugate was then dispensed into each well (100 µL/well). Covered plates were incubated for 60 minutes (±5 min.) at 18-26°C. The solution was then removed and each well washed five times with Wash Solution as described previously. TMB (3,3',5,5' Tetramethylbenzidine) Substrate was then incubated at 18-26°C for 15 minutes (± 1 min., 100 µl/well). Stop Solution (0.31 M maleic acid) was finally added to each well (100 µl/well). The absorbance of the samples and controls was then measured at 450 nm or using a dual wavelength of 450 nm and 650 nm. Sample to positive control signal ratio (S/P %) was then calculated for each sample. The optimal assay cutoff (for tank milk pools of 50 samples) was determined with a ROC (receiver operator characteristic) analysis (positive and negative field samples) and German reference samples (R26 to R29 samples). The cutoffs for the other pool sizes were then derived from the results obtained with the corresponding dilution of a weak positive sample (R29).

**Table 1. Cutoff table. Cutoff is indicated for each pool size: from individual milk to pools of 50 milk samples.**

| | **Pool size** | | | | |
|---|---|---|---|---|---|
| | 1-10 | 11-20 | 21-30 | 31-40 | 41-50 |
| **Negative** | S/P % < 150 | S/P % < 90 | S/P % < 65 | S/P % < 45 | S/P % < 30 |
| **not negative** | S/P % ≥ 150 | S/P % ≥ 90 | S/P % ≥ 65 | S/P % ≥ 45 | S/P % ≥ 30 |

### RESULTS

Comparative evaluation of the gC-DAS-ELISA was performed with negative (IBR-free countries) and positive milk samples from vaccinated and/or infected herds.

Test results were compared to standard commercially available classical biphasic bulk milk indirect ELISA tests which are using whole virus preparations as solid phase antigen.

### Sensitivity

A total number of 244 positive milk samples were tested on the gC-based assay. This sample set included milk samples from individual vaccinated as well as infected cows (BoHV-1 outbreaks) collected from various farms. All animals tested showed detectable anti-gC reactivity in milk. All samples were indeed successfully identified as positive by the gC-assay when tested neat or pre-diluted at a dilution factor of 1:50 **(Table 2).**

**Table 2: Diagnostic sensitivity of the gC-DAS-ELISA (IBR Tank Milk) and two commercially available whole virus biphasic ELISAs. A total of 244 positive sample were tested on gC-DAS-ELISA, and a subset of these samples was also tested on IDEXX BHV-1 Bulk Milk (ELISA 1) and another commercially available test (ELISA 2). 213 (labeled with *) of these 244 samples were also tested in a 1/50 dilution with the gC-DAS-ELISA.**

| | | IBR Tank Milk | | | ELISA 1 | | | ELISA 2 | | |
|---|---|---|---|---|---|---|---|---|---|---|
| sample description | status | n | positive | negative | n | positive | negative | n | positive | negative |
| Netherlands, 2019, group B, individual milks: | vaccinated, not infected | 75 | 75 | 0 | 75 | 75 | 0 | n. a. | n. a. | n. a. |
| Netherlands, 2019, group C, individual milks: | not vaccinated, infected | 42 | 42 | 0 | 42 | 42 | 0 | n. a. | n. a. | n. a. |
| Netherlands, 2019, group D, individual milks: | vaccinated, infected | 98 | 98 | 0 | 98 | 98 | 0 | n. a. | n. a. | n. a. |
| Netherlands, 2019, group B, 1/50* | vaccinated, not infected | 75 | 75 | 0 | n. a. | n. a. | n. a. | n. a. | n. a. | n. a. |
| Netherlands, 2019, group C, 1/50* | not vaccinated, infected | 42 | 42 | 0 | n. a. | n. a. | n. a. | n. a. | n. a. | n. a. |
| Netherlands, 2019, group D, 1/50* | vaccinated, infected | 98 | 98 | 0 | n. a. | n. a. | n.a. | n. a. | n. a. | n. a. |
| France, 1/50* | positive serum | 5 | 5 | 0 | 5 | 5 | 0 | 5 | 5 | 0 |
| US, pools of unknown size | vaccinated | 20 | 20 | 0 | 20 | 20 | 0 | 20 | 20 | 0 |
| Germany, pools of unknown size | positive | 4 | 3 | 1 | 4 | 4 | 0 | 4 | 4 | 0 |
| | total | **459** | 458 | 1 | **244** | 244 | 0 | **29** | 29 | 0 |
| | **sensitivity (%)** | 99.78 | | | 100.00 | | | 100.00 | | |

| | | | | | | | | | | |
|---|---|---|---|---|---|---|---|---|---|---|
| *dilutions were prepared in commercial skimmed milk | | | | | | | | | | |

This high diagnostic sensitivity obtained with the assay demonstrates for the first time that recombinant gC glycoprotein allows efficient detection of antibody-responses in bovine milk samples and can substitute for classical whole virus antigens without compromising overall assay sensitivity.

In addition, analytical sensitivity was examined using serial dilutions of strong positive samples. With this aim in view German Reference Samples and strong positive milk samples from naturally infected cows were tested at various dilutions **(Table 2).**

**Figure 1** shows the analytical sensitivity of the gC-DAS-ELISA according to the invention which is comparable to that of IDEXX BHV-1 Bulk Milk (ELISA 1). The cut-offs for pools of 50 milk samples (pool 50) are indicated by a blue dotted line (IBR Tank Milk) or a red dotted line (ELISA 1), and the cutoff of both assays for individual milk samples is indicated by a purple dotted line. PC, positive control; NC, negative control; R27 (serum)/R29 (milk), German reference samples; SBES, a BoHV-1 antibody positive serum used as positive control for the gC-DAS-ELISA; NL-C1/NL-C13/NL-C36, milk field samples of cows infected with BoHV-1 from the Netherlands.

Thus, detectability thresholds of the gC-based assay according to the invention were comparable to classical tests using whole virus-derived antigens (Fig. 1). The gC-based ELISA also detected anti-BoHV-1 antibodies in serum samples.

In another experiment, diagnostic sensitivity of the gC-DAS-ELISA according to the invention in pools of 100 milk samples was determined. With this aim in view, German Reference Samples and as well as positive milk samples from naturally infected cows were tested at a dilution of 1:100. **Figure 2** shows the results at dilutions of 1:100 in skim milk. The cut-offs for pools of 41 to 50 milk samples are indicated by a dotted line. R28 1:50, R28 1:100, R29 1:50, and R29 1:100 : German reference samples diluted 1:50 or 1:100, respectively; infected 1:100 (DK) and infected 1:100 (NL) : Milk field samples of cows infected with BoHV-1 from Denmark (n=23) and the Netherlands (n=41), respectively. The vast majority of samples were indeed successfully identified as positive by the gC-assay when tested at a dilution factor of 1:100. The sensitivity at a dilution of 1:100 was 98.4% (95% confidence interval: 90.7%-100%)

Altogether, diagnostic and analytical sensitivity data show that gC can be used as a single recombinant antigen instead of whole virus-based antigens without any gap in detection due to "non gC reactors" or weak reactivity.

### Specificity

*Diagnostic specificity I* - Currently available assays are biphasic: They are all using a control well in addition to the BoHV-1 antigen well to remove non-specific signal caused by non-specific binding.

The monophasic gC-based assay demonstrated equivalent to superior specificity as compared to classical biphasic indirect ELISA kits using whole virus antigen preparations **(Table 3).**

**Table 3: Diagnostic specificity (I) of the gC-DAS-ELISA assessed with samples from countries free of BoHV-1. A total of 493 samples were tested with the gC-DAS-ELISA, 158 samples were tested with ELISA 1 and 16 with ELISA 2**

| sample description | IBR Tank Milk | | | ELISA 1 | | | ELISA 2 | | |
|---|---|---|---|---|---|---|---|---|---|
| | N | positive | negative | n | positive | negative | n | positive | negative |
| Switzerland 2019, pools | 107 | 0 | 107 | 7 | 0 | 7 | 7 | 0 | 7 |
| Switzerland 2021, fresh pools | 187 | 0 | 187 | n.a. | n.a. | n.a. | n.a. | n.a. | n.a. |
| Germany 2019, individual | 103 | 0 | 103 | 103 | 2 | 101 | n.a. | n.a. | n.a. |
| Germany 2019, pools | 86 | 0 | 86 | 39 | 1 | 38 | n.a. | n.a. | n.a. |
| Commercial milks 2019 | 9 | 0 | 9 | 9 | 0 | 9 | 9 | 0 | 9 |
| R30 (FLI negative reference) | 1 | 0 | 1 | n.a. | n.a. | n.a. | n.a. | n.a. | n.a. |
| total | **493** | 0 | 493 | **158** | 3 | 155 | **16** | 0 | 16 |
| **specificity (%)** | **100.00** | | | **98.10** | | | **100** | | |
| 95% CI | 99%-100% | | | 94.3%-99.6% | | | 76.9%-100% | | |

Individual and pooled milk samples from IBR-free countries tested all negative on the gC-based ELISA. Samples evaluated included samples from Switzerland (N=294), Germany (N=189) as well as commercial milks (N=9).

*Diagnostic specificity II -* An additional 336 samples had been collected by users of the IDEXX BHV-1 Bulk Milk (ELISA 1) test; these samples also originated from countries free of BoHV-1. A significant portion of these samples had not been tested negative with ELISA 1 by the customers; these non-negatives samples were further characterized by the customers and were found to be negative for anti-BoHV-1 antibodies and were thus referred to as "pseudo-vaccinates" or simply false positive on the ELISA 1. When tested internally, 155 out of these 336 samples (effectively only 334 samples could be run) could be confirmed to not test negative with ELISA 1. When these 336 samples were tested on the gC-DAS-ELISA, only 19 were not negative (Table 4).

**Table 4: Diagnostic specificity (II) of the gC-DAS-ELISA assessed with 336 samples preselected by customers using the IDEXX BHV-1 Bulk Milk test (ELISA 1). Of these samples, 334 samples were tested on ELISA 1 and 41 were tested on ELISA 2.**

| sample description | IBR Tank Milk | | | ELISA 1 | | | ELISA 2 | | |
|---|---|---|---|---|---|---|---|---|---|
| | n | positive | negative | n | positive | negative | n | positive | negative |
| Austria, pools 2019 | 100 | 0 | 100 | 99 | 20 | 79 | n. a. | n. a. | n. a. |
| Germany, pools | 16 | 0 | 16 | 15 | 13 | 2 | 15 | 4 | 11 |
| Germany WP164, pools | 26 | 0 | 26 | 26 | 19 | 7 | 26 | 16 | 10 |
| Germany 2020, pools | 28 | 4 | 24 | 28 | 5 | 23 | n. a. | n. a. | n. a. |
| Germany 2019; plasma diluted 1/50 in skimmed milk | 166 | 15 | 151 | 166 | 98 | 68 | n. a. | n. a. | n. a. |
| total | **336** | 19 | 317 | **334** | 155 | 179 | **41** | 20 | 21 |
| specificity (%) | **94.35** | | | **53.59** | | | **51.22** | | |
| 95% CI | 91.3%-96.4% | | | 48.2%-58.9% | | | 36.5%-65.7% | | |

### Conclusion

It could be shown that the use of a single recombinant protein according to the disclosure instead of whole virus preparations, and preferably the use of a DAS assay format, can also be considered to contribute to an advantageous performance and to the higher specificity of this DAS ELISA. Using the same gC-coated plates the specificity of a gC-based DAS assay was indeed higher compared to an indirect ELISA using anti-bovine IgG conjugate as detector.

### Example 2

### N-terminus of BoHV-I glycoprotein C - Cloning, purification, and assessment of its suitability as antigen in an ELISA for detection of anti BoHV-I antibodies in bovine milk

### Summary:

The N-terminal region of glycoprotein c (gC-Nterm) of bovine herpesvirus 1 (BoHV-1) was recombinantly produced in *E. coli* as a fusion protein with a maltose binding protein (MBP) and an Avi-tag at its N- and C-terminus, respectively. The biotinylated protein was immobilized on microtiter plates and tested in an ELISA to assess reactivity with anti-BoHV-1 antibodies (Ab) present in bovine milk. The ELISA showed a strong signal for two BoHV-1 Ab positive milk samples and no signal for a BoHV-1 Ab negative milk demonstrating the antigenicity of gC-Nterm and its suitability as antigen in an ELISA for detection of BoHV-1 antibodies.

### Materials and methods:

*Cloning and purification of gC-Nterm* - The DNA sequence of BoHV-1 gC (accession NP 045314.1) corresponding to amino acids Ala26-Leu141 plus a C-terminal amino acid stretch with the sequence GGSGGSSGLNDIFEAQKIEWHE i.e. GS linker + AviTag (Avidity, Aurora, USA) was cloned into the vector pMal-p5X (NEB, Ipswich, USA) and the resulting vector was used to transform E. coli C3029 (NEB, Ipswich, USA) x pBirAcm (Avidity, Aurora, USA). The resulting strain was grown in LB supplemented with 100 ug/ml ampicillin and 10 ug/ml chloramphenicol at 37 °C for 2 h. Biotin was added to a final concentration of 50 uM. Expression was induced by addition of 100 uM IPTG and the culture was further grown for 4 h at 30 °C. Cell were pelleted by centrifugation at 4500 g for 15 min at 4 °C, resuspended in 3 ml column buffer A (20 mM Tris-Cl, 1 mM EDTA, 150 mM NaCl, pH 8) per g wet cells in the presence of protease inhibitor (complete EDTA-free; Roche, Basel, Switzerland) and 50 ug/ml DNase I. Cells were lysed by passing the suspension twice through a Microfluidizer LV1 (Microfluidics, Westwood, USA) at 30000 psi. The lysate was centrifuged at 100000 g for 1 h at 4 °C and the supernatant was filtered through 0.2 um. The supernatant was loaded onto amylose resin (NEB) previously equilibrated with column buffer A. The resin was washed twice with 2 CV column buffer A and bound protein was eluted with 5 CV column buffer B (column buffer A + 10 mM maltose). The eluate was pooled and subjected to digestion with factor Xa in the presence of 4 mM CaCl₂ for 1 h at room temperature (RT). The degree of biotinylation was assessed by a streptavidin gel-shift assay. The digestion was subjected to anion exchange chromatography using a Toyopearl Minichrom NH2-750F column (Tosoh Bioscience GmbH, Griesheim, Germany) equilibrated with column buffer A; protein bound to the resin was eluted by developing a linear gradient from 0-100% column buffer B (column buffer A containing 2 M NaCl). The peak containing gC-Nterm contained also MBP. The peak fraction was collected and subjected to amylose resin chromatography to remove MBP. The flow through was collected, concentrated and run over a Superdex 75 pg 16/600 gel filtration column (Cytiva, Marlborough, USA) equilibrated with column buffer A. Fractions containing gC-Nterm were pooled and concentrated using a Amicon Ultra spin concentrator (Millipore, Burlington, USA) with a molecular weight cutoff of 3 kDa. The concentration of gC-Nterm was determined spectrophotometrically using an extinction coefficient of 11000 M-1 cm-1 at 280 nm.

*ELISA* - 96 well Maxisorp microtiter plates (Thermo, Waltham, USA) were coated with 100 ul/well of gC-Nterm at a concentration from 7.8 ng/ml - 500 ng/ml in 0.1 M Na-carbonate, pH 9.5 at 4 °C for 14-18 h. Plates were washed five times with 350 ul/well of PBS+0,1% Tween 20. Plates were dried for 2 h at 28 °C, 28% relative humidity. Where applicable, samples were diluted in commercial skimmed milk containing <0.1% fat. Samples (50 ul) were diluted in well 1:2 with 1% non-ionic detergent and this was incubated for 2 h in a humid chamber at RT. Plates were washed five times with 350 ul/well PBS+0.1% Tween20. 100 ul/well of 2.5 ug/ml goat anti-bovine IgG-HRP conjugate diluted in a proprietary buffer were added to each well and incubated for 1 h at RT. Plates were washed five times with 350 ul/well of PBS+0,1% Tween 20, and 100 ul/well of a proprietary tetramethyl benzidine (TMB) solution were added followed by incubation for 15 min at RT. The reaction was stopped by addition of a proprietary acid stop solution. The OD at 450 nm was recorded with a spectrophotometer and corrected by subtraction of the OD at 650 nm.

### Results

The N-terminus of BoHV-1 gC was recombinantly produced in *E. coli* and analyzed by SDS PAGE. gC-Nterm has a theoretical molecular weight of 13889 Da but migrated at approximately 25 kDa. This unusual electrophoretic mobility can be attributed to the large number of prolines (i.e. approx. 23% of the amino acid of the gC N-terminus are prolines) and negatively charged residues (Asp, Glu) present in the amino acid sequence of gC-Nterm. The bands at ~32 kDa and ~17 kDa correspond to the two subunits of factor Xa which was co-purified (Fig. 3).

gC-Nterm was used as antigen in an indirect ELISA for detection of BoHV-**1** antibodies in bovine milk samples. Two BoHV-1 Ab positive samples and one negative sample were tested with the gC-Nterm ELISA. The positive sample EFI (1:1000), a BoHV-1 Ab positive serum diluted 1000-fold in commercial skimmed milk, reacted strongly; at a gC-Nterm coating concentration of 62.5 ng/ml or higher, OD plateaued at OD450=2.2. The other positive samples R28 (1:50), a BoHV-1 Ab positive milk diluted 50-fold in commercial skimmed milk reacted to a lesser extent; at a gC-Nterm coating concentration of 7.8 ng/ml or higher, OD plateaued at OD450=0.5 (Fig. 4).

R28 is a German reference sample with a low Ab titer. According to German regulations, 50-fold diluted R28 should test positive commercial ELISA tests used for screening of tank milks consisting of up to 50 individual milks. The negative milk sample did not generate positive signal over the whole concentration range of gC-Nterm, with the exception that some signal was observed at a gC-Nterm concentration of 31.25 ng/ml which was considered an artifact (Fig. 4).

The results demonstrate that the N-terminus of gC represents a good antigen for the detection of BoHV-1 antibodies in bovine milk.

### Example 3

### Epitope mapping of BoHV-1 gC

### Summary:

Glycoprotein C (gC) of bovine herpesvirus 1 (BoHV-1) is used as antigen and detector in the IDEXX IBR Tank Milk ELISA kit. The goal of the present study was to determine the most immunogenic regions of gC by epitope mapping using a peptide array consisting of biotinylated 15-mer peptides spanning the soluble domain of gC (Ala26-Ser474). Adjacent peptides had an overlap of 11 amino acids (aa). Purified IgG from milk (n=7) or serum (n=3) obtained from BoHV-1 antibody (Ab) positive cows was reacted with each peptide immobilized on a 96-well microtiter plate previously coated with streptavidin. Bovine IgG bound to peptides was detected by incubation with a secondary goat anti-bovine IgG antibody coupled to horseradish peroxidase (HRP). As discussed below, a total of 15 peptides were bound by IgG, and they were distributed over the entire sequence of the soluble domain of gC; they were either bound by only milk IgG samples (n=6), only serum IgG samples (n=6) or both sample types (n=3). The results suggested that the soluble domain of BoHV-1 gC contains multiple important epitopes recognized by bovine IgG in milk and/or serum.

### Materials and methods:

### Purification of IgG:

Bovine serum was mixed with an equal volume of binding buffer (200 mM NaPi, 2 M NaCl, pH 7.4). Diluted bovine serum and milk samples were spun at 100 k g for 1 h at 4 °C. The supernatant of the serum samples and the serum-phase of the milk samples were obtained and filtered through 0.2 um. Resulting samples were loaded onto PROchieveA resin (Avantor, Radnor, PA, USA) equilibrated with 10 CV binding buffer. The column was washed with 20 CV binding buffer and 10 CV elution buffer pH 7 (0.2 M phosphate-citrate buffer, 3 M NaCl, pH 7), and bound IgG was eluted with 5 CV elution buffer pH 3 (0.2 M phosphate-citrate buffer, 3 M NaCl, pH 3). IgG containing fractions were neutralized by addition of ¼ vol. of 1 M Tris-HCl, pH 8. IgG concentration was determined spectrophotometrically using an extinction coefficient of 210000 M-1 cm-1 at 280 nm. Purity of IgG preparations was checked by SDS-PAGE.

### Peptide array:

The amino acid sequence of the soluble domain of BoHV-1 gC (accession NP_045314) i.e. the full length sequence without signal peptide and transmembrane domain (Ala26-Ser474), was used as the template for the synthesis of a peptide array consisting of 15-mers spanning the entire 494 aa such that adjacent peptide sequences overlapped by 11 aa. Peptides (n=110) were synthesized at 2.5 umol scale by Vivitide (Gardner, MA, USA) with an LC-biotin at the N-terminus and an amide group at the C-terminus (Table 1). Lyophilized peptides were dissolved in dimethyl sulfoxide (DMSO) to yield a concentration of 2 mg/ml.

### Epitope mapping ELISA:

Peptides were diluted to 0.1 ug/ml in phosphate buffered saline (PBS) and 100 ul of each peptide were added to the wells of streptavidin coated 96-well microtiter plates previously hydrated with 300 ul double distilled water (ddH2O). As control, the biotinylated soluble domain of gC (gC-bio) was coated at 0.01 uM or 0.005 uM. Plates were incubated for 90 min at RT, washed twice with 400 ul/well PBS + 0.1% Tween20 (PBST), and 100 ul/well of 1 ug/ml purified IgG in PBST were added to each well followed by incubation at RT for 1 h. Plates were washed five times with 400 ul/well PBST and 100 ul/well of 1 ug/ml goat anti-bovine IgG-HRP diluted in a proprietary buffer were added and incubated for 1 h at RT. Plates were washed again five times with 400 ul/well PBST and 100 ul/well of TMB solution were added, followed by incubation for 10 min at RT. The reaction was stopped by addition of 100 ul/well acid stop solution. Plates were read at 450 nm. A cutoff of OD_{450 nm}=0.3 was used to discriminate specific and unspecific signals. After samples had been tested with all peptides in single replicates, peptide hits were confirmed by testing samples in duplicate on each peptide identified in the screening.

**Table 1: List of peptides (n=110) spanning the amino acid sequence Ala26-S474 of BoHV-1 gC:**

| **Peptide no.** | **Sequence** | **Length** | **SEQ ID No.** |
|---|---|---|---|
| 1 | AEEAEASPSPPPSPS | 15 | 6 |
| 2 | EASPSPPPSPSPTET | 15 | 7 |
| 3 | SPPPSPSPTETESSA | 15 | 8 |
| 4 | SPSPTETESSAGTTG | 15 | 9 |
| 5 | TETESSAGTTGATPP | 15 | 10 |
| 6 | SSAGTTGATPPTPNS | 15 | 11 |
| 7 | TTGATPPTPNSPDAT | 15 | 12 |
| 8 | TPPTPNSPDATPEDS | 15 | 13 |
| 9 | PNSPDATPEDSTPGA | 15 | 14 |
| 10 | DATPEDSTPGATTPV | 15 | 15 |
| 11 | EDSTPGATTPVGTPE | 15 | 16 |
| 12 | PGATTPVGTPEPPSV | 15 | 17 |
| 13 | TPVGTPEPPSVSEHD | 15 | 18 |
| 14 | TPEPPSVSEHDPPVT | 15 | 19 |
| 15 | PSVSEHDPPVTNSTP | 15 | 20 |
| 16 | EHDPPVTNSTPPPAP | 15 | 21 |
| 17 | PVTNSTPPPAPPEDG | 15 | 22 |
| 18 | STPPPAPPEDGRPGG | 15 | 23 |
| 19 | PAPPEDGRPGGAGNA | 15 | 24 |
| 20 | EDGRPGGAGNASRDG | 15 | 25 |
| 21 | PGGAGNASRDGRPSG | 15 | 26 |
| 22 | GNASRDGRPSGGGRP | 15 | 27 |
| 23 | RDGRPSGGGRPRPPR | 15 | 28 |
| 24 | PSGGGRPRPPRPSKA | 15 | 29 |
| 25 | GRPRPPRPSKAPPKE | 15 | 30 |
| 26 | PPRPSKAPPKERKWM | 15 | 31 |
| 27 | SKAPPKERKWMLCER | 15 | 32 |
| 28 | PKERKWMLCEREAVA | 15 | 33 |
| 29 | KWMLCEREAVAASYA | 15 | 34 |
| 30 | CEREAVAASYAEPLY | 15 | 35 |
| 31 | AVAASYAEPLYVHCG | 15 | 36 |
| 32 | SYAEPLYVHCGVADN | 15 | 37 |
| 33 | PLYVHCGVADNATGG | 15 | 38 |
| 34 | HCGVADNATGGARLE | 15 | 39 |
| 35 | ADNATGGARLELWFQ | 15 | 40 |
| 36 | TGGARLELWFQRVGR | 15 | 41 |
| 37 | RLELWFQRVGRFRST | 15 | 42 |
| 38 | WFQRVGRFRSTRGDD | 15 | 43 |
| 39 | VGRFRSTRGDDEAVR | 15 | 44 |
| 40 | RSTRGDDEAVRNPFP | 15 | 45 |
| 41 | GDDEAVRNPFPRAPP | 15 | 46 |
| 42 | AVRNPFPRAPPVLLF | 15 | 47 |
| 43 | PFPRAPPVLLFVAQN | 15 | 48 |
| 44 | APPVLLFVAQNGSIA | 15 | 49 |
| 45 | LLFVAQNGSIAYRSA | 15 | 50 |
| 46 | AQNGSIAYRSAELGD | 15 | 51 |
| 47 | SIAYRSAELGDNYIF | 15 | 52 |
| 48 | RSAELGDNYIFPSPA | 15 | 53 |
| 49 | LGDNYIFPSPADPRN | 15 | 54 |
| 50 | YIFPSPADPRNLPLT | 15 | 55 |
| 51 | SPADPRNLPLTVRSL | 15 | 56 |
| 52 | PRNLPLTVRSLTAAT | 15 | 57 |
| 53 | PLTVRSLTAATEGVY | 15 | 58 |
| 54 | RSLTAATEGVYTWRR | 15 | 59 |
| 55 | AATEGVYTWRRDMGT | 15 | 60 |
| 56 | GVYTWRRDMGTKSQ R | 15 | 61 |
| 57 | WRRDMGTKSQRKVVT | 15 | 62 |
| 58 | MGTKSQRKVVTVTTH | 15 | 63 |
| 59 | SQRKVVTVTTHRAPA | 15 | 64 |
| 60 | VVTVTTHRAPAVSVE | 15 | 65 |
| 61 | TTHRAPAVSVEPQPA | 15 | 66 |
| 62 | APAVSVEPQPALEGA | 15 | 67 |
| 63 | SVEPQPALEGAGYAA | 15 | 68 |
| 64 | QPALEGAGYAAVCRA | 15 | 69 |
| 65 | EGAGYAAVCRAAEYY | 15 | 70 |
| 66 | YAAVCRAAEYYPPRS | 15 | 71 |
| 67 | CRAAEYYPPRSTRLH | 15 | 72 |
| 68 | EYYPPRSTRLHWFRN | 15 | 73 |
| 69 | PRSTRLHWFRNGYPV | 15 | 74 |
| 70 | RLHWFRNGYPVEARH | 15 | 75 |
| 71 | FRNGYPVEARHARDV | 15 | 76 |
| 72 | YPVEARHARDVFTVD | 15 | 77 |
| 73 | ARHARDVFTVDDSGL | 15 | 78 |
| 74 | RDVFTVDDSGLFSRT | 15 | 79 |
| 75 | TVDDSGLFSRTSVLT | 15 | 80 |
| 76 | SGLFSRTSVLTLEDA | 15 | 81 |
| 77 | SRTSVLTLEDATPTA | 15 | 82 |
| 78 | VLTLEDATPTAHPPN | 15 | 83 |
| 79 | EDATPTAHPPNLRCD | 15 | 84 |
| 80 | PTAHPPNLRCDVSWF | 15 | 85 |
| 81 | PPNLRCDVSWFQSAN | 15 | 86 |
| 82 | RCDVSWFQSANMERR | 15 | 87 |
| 83 | SWFQSANMERRFYAA | 15 | 88 |
| 84 | SANMERRFYAAGTPA | 15 | 89 |
| 85 | ERRFYAAGTPAVYRP | 15 | 90 |
| 86 | YAAGTPAVYRPPELR | 15 | 91 |
| 87 | TPAVYRPPELRVYFE | 15 | 92 |
| 88 | YRPPELRVYFEGGEA | 15 | 93 |
| 89 | ELRVYFEGGEAVCEA | 15 | 94 |
| 90 | YFEGGEAVCEARCVP | 15 | 95 |
| 91 | GEAVCEARCVPEGRV | 15 | 96 |
| 92 | CEARCVPEGRVSLRW | 15 | 97 |
| 93 | CVPEGRVSLRWTVRD | 15 | 98 |
| 94 | GRVSLRWTVRDGIAP | 15 | 99 |
| 95 | LRWTVRDGIAPSRTE | 15 | 100 |
| 96 | VRDGIAPSRTEQTGV | 15 | 101 |
| 97 | IAPSRTEQTGVCAER | 15 | 102 |
| 98 | RTEQTGVCAERPGLV | 15 | 103 |
| 99 | TGVCAERPGLVNLRG | 15 | 104 |
| 100 | AERPGLVNLRGVRLL | 15 | 105 |
| 101 | GLVNLRGVRLLSTTD | 15 | 106 |
| 102 | LRGVRLLSTTDGPVD | 15 | 107 |
| 103 | RLLSTTDGPVDYTCT | 15 | 108 |
| 104 | TTDGPVDYTCTATGY | 15 | 109 |
| 105 | PVDYTCTATGYPAPL | 15 | 110 |
| 106 | TCTATGYPAPLPEFS | 15 | 111 |
| 107 | TGYPAPLPEFSATAT | 15 | 112 |
| 108 | APLPEFSATATYDAS | 15 | 113 |
| 109 | EFSATATYDASPGLI | 15 | 114 |
| 110 | TATYDASPGLIGS | 13 | 115 |

### Results:

The presence or absence of anti-BoHV-1 antibodies in bovine milk and serum samples had previously been determined with commercially available ELISA tests (not shown). IgG was purified from BoHV-1 antibody positive bovine milk (n=7) and serum (n=3) serum samples. In addition, IgG was purified from two BoHV-1 negative milk samples. Purity of all IgG preparations was at least 95% as judged by SDS-PAGE (not shown).

IgG samples were reacted with each peptide of a 15-mer gC peptide array immobilized on a 96-well microtiter plate. All IgG preparations from BoHV-1 Ab positive samples bound to recombinant gC and to at least one peptide of the gC peptide array, as indicated by an OD450>0.3. IgG from negative samples did neither react with gC nor with any peptide (Fig. 5).

In a second step, each IgG sample was tested in duplicate with each peptide that gave a positive result with any sample in the screening ELISA. Out of the ten IgG preparations from BoHV-1 Ab positive samples (7 milk and 3 serum samples), peptide binding was confirmed for 8 samples. In total, 15 peptides of the array were recognized by the IgG samples. Out of these 15 peptides, 6 peptides were recognized exclusively by milk IgG (peptide 7, 15, 18, 23, 103, 104), 6 were recognized by serum IgG (peptides 13, 41, 64, 66, 79, 109) and 3 were recognized by milk and serum IgG (peptides 8, 14, 110) (Fig. 6, Table 2+3). The above results demonstrate that epitopes that are recognized by IgG from BoHV-1 Ab positive milk and serum samples are distributed along the entire sequence of the soluble domain of gC (A26-S474).

**Table 2: Results of the BoHV-1 gC epitope mapping. Numbers in brackets indicate the number of the peptide(s) bound by the respective sample.**

| gC sequence covered by detected peptide(s) | Peptide no. | sample | |
|---|---|---|---|
| | | milk TqG | serum TqG |
| T50-S68 | 7, 8 | DK-E1 (7,8), DK-E17 (7) | FCS (8) |
| T74-G108 | 13-15, 18 | DK-E11 (14, 15, 18), DK-E17 (14, 18), DK-E21 (14, 15) | 2166/97 (13, 14) |
| R114-R128 | 23 | DK-E11, DK-E21 | |
| G186-P200 | 41 | | #897 |
| Q278-S300 | 64, 66 | | #897 (64, 66), 2166/97 (64) |
| E338-D352 | 79 | | #897 |
| R434-Y452 | 103-104 | DK-E1 (103, 104) | |
| E458-S474 | 109-110 | DK-E11 (110), DK-E17 (110), DK-E21 (110) | 2166/97 (109, 110) |

**Table 3: List of BoHV-1 gC peptides that IgG preparations from BoHV-1 Ab positive sera and milks reacted with.**

| **Peptide no.** | **Sequence** | **Length** |
|---|---|---|
| 7 | TTGATPPTPNSPDAT | 15 |
| 8 | TPPTPNSPDATPEDS | 15 |
| 13 | TPVGTPEPPSVSEHD | 15 |
| 14 | TPEPPSVSEHDPPVT | 15 |
| 15 | PSVSEHDPPVTNSTP | 15 |
| 18 | STPPPAPPEDGRPGG | 15 |
| 23 | RDGRPSGGGRPRPPR | 15 |
| 41 | GDDEAVRNPFPRAPP | 15 |
| 64 | QPALEGAGYAAVCRA | 15 |
| 66 | YAAVCRAAEYYPPRS | 15 |
| 79 | EDATPTAHPPNLRCD | 15 |
| 103 | RLLSTTDGPVDYTCT | 15 |
| 104 | TTDGPVDYTCTATGY | 15 |
| 109 | EFSATATYDASPGLI | 15 |
| 110 | TATYDASPGLIGS | 13 |

**Table 4: List of epitopes deduced from overlaps of certain BoHV-1 gC peptides shown in Table 3.**

| **Overlapping Peptide nos.** | **Overlapping Sequence** | **Length** | **SEQ ID No.** |
|---|---|---|---|
| 7 & 8 | TPPTPNSPDAT | 11 | 116 |
| 13 & 14 & 15 | PSVSEHD | 7 | 117 |
| 64 & 66 | YAAVCRA | 7 | 118 |
| 103 & 104 | TTDGPVDYTCT | 11 | 119 |
| 109 & 110 | TATYDASPGLI | 11 | 120 |

**Table 5: Peptide sequence variants.**

| **Variant Peptide no.** | **Sequence** | **Length** | **SEQ ID No.** |
|---|---|---|---|
| 7-v | TTGXTPPTPNSPDAT | 15 | 121 |
| 13-v | TPXGTPEPPSVSEHD | 15 | 122 |
| 23-v | RDGRPSGGGRPRPPX | 15 | 123 |
| 64-v | XPALXGAGYAAVCRA | 15 | 124 |
| 66-v | YAAVCRAAXYYPPRS | 15 | 125 |
| 109-v | EFSATATYDASPGLX | 15 | 126 |
| 110-v | TATYDASPGLXGS | 13 | 127 |
| 109110-v | TATYDASPGLX | 11 | 128 |

Table 5 variants:
The X at position 4 of SEQ ID NO: 121 stands for A or T.
The X at position 3 of SEQ ID NO: 122 stands for V or G.
The X at position 15 of SEQ ID NO: 123 stands for R or Q.
The X at position 1 of SEQ ID NO: 124 stands for Q or R.
The X at position 5 of SEQ ID NO: 124 stands for E or Q.
The X at position 9 of SEQ ID NO: 125 stands for E or K.
The X at position 15 of SEQ ID NO: 126 stands for I or V.
The X at position 11 of SEQ ID NO: 127 stands for I or V.
The X at position 11 of SEQ ID NO: 128 stands for I or V.

Further aspects are described below:
One aspect of the disclosure is an isolated polypeptide sequence comprising or consisting of SEQ ID No. 3 or 4, or any of SEQ ID Nos. 6 to 128 or a combination of any thereof or a fragment thereof having at least 90% sequence identity with SEQ ID Nos. 3 or 4, or any of SEQ ID Nos. 6 to 128 , preferably at least 95%, more preferably at least 98% sequence identity, or consisting of at least 15 contiguous amino acids of an amino acid sequence set forth as SEQ ID NOs. 3 or 4, or any of SEQ ID Nos. 6 to 128.

One aspect of the disclosure is an isolated polypeptide as described herein selected from the group consisting of the sequences SEQ ID Nos. 3 or 4, or any of SEQ ID Nos. 6 to 128 or a combination of any thereof.

One aspect of the disclosure is an isolated polypeptide as described herein wherein the polypeptide is viral, an isolated polypeptide as described herein wherein the polypeptide is derived from Bovine Herpes Virus Type 1, preferably
an isolated polypeptide as described herein wherein the polypeptide is produced in prokaryotic or eukaryotic cells, preferably in CHO or Expi293 or HEK cells.

One aspect of the disclosure is an isolated polypeptide as described herein wherein the polypeptide comprises an antigenic determinant that is recognized by antibodies specific for BoHV-1.

One aspect of the disclosure is an isolated polypeptide as described herein wherein the one or more isolated polypeptides are linked to an indicator reagent, a signal sequence, a stop transfer sequence, an amino acid spacer, a transmembrane domain, a protein purification ligand, a heterologous polypeptide, a moiety that enhances an immune response, a moiety that facilitates purification, a moiety that facilitates polypeptide stability, one or more additional polypeptides as described herein or a combination thereof.

One aspect of the disclosure is a method of identifying in a sample a non-human animal infected with BoHV-1 comprising the steps:
i. contacting a sample with a polypeptide as described herein under conditions that a complex of an antibody specific against BHV-1 in the sample and the polypeptide can form;
ii. detecting the complex;
wherein the detection of the complex is indicative of an infection with BoHV-1.

One aspect of the disclosure is a method as described herein wherein the complex is detected by labeling said complex with a detectable agent, preferably a color reactant, or a fluorescent agent.

One aspect of the disclosure is a method as described herein wherein the detectable agent is conjugated to a polypeptide as described herein.

One aspect of the disclosure is a method as described herein, wherein the detectable agent is selected from biotin*SA-polyHRP20, an enzyme, a fluorophore, or a colorimetric agent.

One aspect of the disclosure is a method as described herein, wherein the polypeptide is immobilized on a solid support.

One aspect of the disclosure is a method as described herein wherein the method is an ELISA (enzyme linked immune-sorbent assay), preferably a multi-step or single-step method, preferably
wherein the ELISA is monophasic.

One aspect of the disclosure is a method as described herein wherein the sample is whole milk or tank milk, preferably
wherein the sample is a pool of milk from a plurality of cows, preferably from 50 cows or 100 cows, preferably
wherein the sample is whole blood, serum or plasma.

One aspect of the disclosure is a kit for the detection of a BoHV-1 infection in a sample comprising:
i. a polypeptides as described herein; and
ii. a means for detection of a complex formed between the polypeptide and an antibody specific against BoHV-1.

One aspect of the disclosure is a method for making or/and isolating a polypeptide as described herein, comprising the steps of i. expressing the polypeptide in prokaryotic or eukaryotic call, preferably Expi293 cells, ii. desalting, iii. one or more ion-exchange, and iv. one or more size exclusion steps.

One aspect of the disclosure is a method as described herein further comprising treating the non-human animal with one or more antivirals, one or more non-steroidal anti-inflammatories, and/or one or more antibiotics if a complex is detected in the sample.

One aspect of the disclosure is a nucleic acid sequence encoding a polypeptide as set forth in any of SEQ ID NOs. 3, 4 or 5, or 6 to 128, or a nucleic acid sequence encoding a polypeptide as set forth herein.

### Sequences

The following sequences are of special interest for the disclosure of the invention. It is one preferred aspect of the invention to use a gC protein, or fragment thereof or a polypeptide derived therefrom which is particularly useful in the method and kit of the disclosure.
>gC full-length [= accession NP_045314.1] 
MGPLGRAWLIAAIFAWALLSARRGLAEEAEASPSPPPSPSPTETESSAGTTGATPPT
PNSPDATPEDSTPGATTPVGTPEPPSVSEHDPPVTNSTPPPAPPEDGRPGGAGNAS
RDGRPSGGGRPRPPRPSKAPPKERKWMLCEREAVAASYAEPLYVHCGVADNATG
GARLELWFQRVGRFRSTRGDDEAVRNPFPRAPPVLLFVAQNGSIAYRSAELGDNYI
FPSPADPRNLPLTVRSLTAATEGVYTWRRDMGTKSQRKVVTVTTHRAPAVSVEPQ
PALEGAGYAAVCRAAEYYPPRSTRLHWFRNGYPVEARHARDVFTVDDSGLFSRTS
VLTLEDATPTAHPPNLRCDVSWFQSANMERRFYAAGTPAVYRPPELRVYFEGGEAV
CEARCVPEGRVSLRWTVRDGIAPSRTEQTGVCAERPGLVNLRGVRLLSTTDGPVD
YTCTATGYPAPLPEFSATATYDASPGLIGSPVLVSVVAVACGLGAVGLLLVAASCLR
RKARARL
 >gC full-length with variants 
MGPLGRAWLIAAIFAWALLSARRGLAEEAEASPSPPPSPXPTETESSAGTTGXTPPT
PNSPDATPEDSTPGATTPXGTPEPPSVSEHDPPVTNSTPPPAPPEDGRPGGAGNAS
RDGRPSGGGRPRPPXPSKAPPKXRXWMLCXREAVAASYAEPLYVHCGVADNATG
XARLELWFQRVGRFRSTRGDDEAVRNPFPRAPPVLLFXXQNGSIAYRSAELGXNYI
FPSPADPRNLPLTVXSLTAATEGVYTWRRDMGXKSQRKVVTVTTHRAPAVSVEPXP
ALXGAGYAAVCRAAXYYPPRSTRLXWFRNGYPVEARHARDVFTVDDSGLFSRTSV
LTLEDATPTAHPPNLRCDVSWXQSANMERRFYAAGTPAVYRPPELRVYFEGGEAVC
EARCVPXGRVSLRWTVRDGIAPSRTXQTGXCAERPGLVNLRGVRLLSTTDGPVDY
TCTATGYPAPLPEFSATATYDASPGLXGSPVLVSVVAVACGLGAVGLLLVAASCLRR
KARARL
Wherein the X at position 40 is S or C
Wherein the X at position 53 is A or T
Wherein the X at position 76 is V or G
Wherein the X at position 128 is R or Q
Wherein the X at position 136 is E or G
Wherein the X at position 138 is K or E
Wherein the X at position 143 is E or K
Wherein the X at position 168 is G or N
Wherein the X at position 205 is V or A
Wherein the X at position 206 is A or V
Wherein the X at position 220 is D or G
Wherein the X at position 238 is R or C
Wherein the X at position 256 is T or A
Wherein the X at position 278 is Q or R
Wherein the X at position 282 is E or Q
Wherein the X at position 294 is E or K
Wherein the X at position 304 is H or R
Wherein the X at position 356 is F or Y
Wherein the X at position 397 is E or G
Wherein the X at position 416 is E or G
Wherein the X at position 420 is V or A
Wherein the X at position 472 is I or V
>gC soluble domain [=Ala26-Ser474 of NP_045314.1] 
AEEAEASPSPPPSPSPTETESSAGTTGATPPTPNSPDATPEDSTPGATTPVGTPEPP
SVSEHDPPVTNSTPPPAPPEDGRPGGAGNASRDGRPSGGRPRPPRPSKAPPKER
KWMLCEREAVAASYAEPLYVHCGVADNATGGARLELWFQRVGRFRSTRGDDEAV
RNPFPRAPPVLLFVAQNGSIAYRSAELGDNYIFPSPADPRNLPLTVRSLTAATEGVYT
WRRDMGTKSQRKVVTVTTHRAPAVSVEPQPALEGAGYAAVCRAAEYYPPRSTRLH
WFRNGYPVEARHARDVFTVDDSGLFSRTSVLTLEDATPTAHPPNLRCDVSWFQSA
NMERRFYAAGTPAVYRPPELRVYFEGGEAVCEARCVPEGRVSLRWTVRDGIAPSRT
EQTGVCAERPGLVNLRGVRLLSTTDGPVDYTCTATGYPAPLPEFSATATYDASPGLI
GS
 >gC soluble domain with variants 
AEEAEASPSPPPSPXPTETESSAGTTGXTPPTPNSPDATPEDSTPGATTPXGTPEPP
SVSEHDPPVTNSTPPPAPPEDGRPGGAGNASRDGRPSGGRPRPPXPSKAPPKXR
XWMLCXREAVAASYAEPLYVHCGVADNATGXARLELWFQRVGRFRSTRGDDEAV
 
RNPFPRAPPVLLFXXQNGSIAYRSAELGXNYIFPSPADPRNLPLTVXSLTAATEGVYT
WRRDMGXKSQRKVVTVTTHRAPAVSVEPXPALXGAGYAAVCRAAXYYPPRSTRLX
WFRNGYPVEARHARDVFTVDDSGLFSRTSVLTLEDATPTAHPPNLRCDVSWXQSA
NMERRFYAAGTPAVYRPPELRVYFEGGEAVCEARCVPXGRVSLRWTVRDGIAPSRT
XQTGXCAERPGLVNLRGVRLLSTTDGPVDYTCTATGYPAPLPEFSATATYDASPGL
XGS
Wherein the X at position 15 is S or C
Wherein the X at position 28 is A or T
Wherein the X at position 51 is V or G
Wherein the X at position 103 is R or Q
Wherein the X at position 111 is E or G
Wherein the X at position 113 is K or E
Wherein the X at position 118 is E or K
Wherein the X at position 143 is G or N
Wherein the X at position 180 is V or A
Wherein the X at position 181 is A or V
Wherein the X at position 195 is D or G
Wherein the X at position 213 is R or C
Wherein the X at position 231 is T or A
Wherein the X at position 253 is Q or R
Wherein the X at position 257 is E or Q
Wherein the X at position 269 is E or K
Wherein the X at position 279 is H or R
Wherein the X at position 331 is F or Y
Wherein the X at position 372 is E or G
Wherein the X at position 391 is E or G
Wherein the X at position 395 is V or A
Wherein the X at position 447 is I or V
>gC soluble domain with signal sequence, tags, linkers [pHA25401]

METDTLLLWVLLLWVPGSTGDAAQPARRAEEAEASPSPPPSPSPTETESSAGTTGA
TPPTPNSPDATPEDSTPGATTPVGTPEPPSVSEHDPPVTNSTPPPAPPEDGRPGGA
GNASRDGRPSGGGRPRPPRPSKAPPKERKWMLCEREAVAASYAEPLYVHCGVAD
NATGGARLELWFQRVGRFRSTRGDDEAVRNPFPRAPPVLLFVAQNGSIAYRSAEL
GDNYIFPSPADPRNLPLTVRSLTAATEGVYTWRRDMGTKSQRKVVTVTTHRAPAV
SVEPQPALEGAGYAAVCRAAEYYPPRSTRLHWFRNGYPVEARHARDVFTVDDSGL
FSRTSVLTLEDATPTAHPPNLRCDVSWFQSANMERRFYAAGTPAVYRPPELRVYFE
GGEAVCEARCVPEGRVSLRWTVRDGIAPSRTEQTGVCAERPGLVNLRGVRLLSTT
DGPVDYTCTATGYPAPLPEFSATATYDASPGLIGSGGSGGSSGLNDIFEAQKIEWH
EGSHHHHHH

| **Advantageous Peptide no.** | **Sequence** | **Length** |
|---|---|---|
| 7 | TTGATPPTPNSPDAT | 15 |
| 8 | TPPTPNSPDATPEDS | 15 |
| 13 | TPVGTPEPPSVSEHD | 15 |
| 14 | TPEPPSVSEHDPPVT | 15 |
| 15 | PSVSEHDPPVTNSTP | 15 |
| 18 | STPPPAPPEDGRPGG | 15 |
| 23 | RDGRPSGGGRPRPPR | 15 |
| 41 | GDDEAVRNPFPRAPP | 15 |
| 64 | QPALEGAGYAAVCRA | 15 |
| 66 | YAAVCRAAEYYPPRS | 15 |
| 79 | EDATPTAHPPNLRCD | 15 |
| 103 | RLLSTTDGPVDYTCT | 15 |
| 104 | TTDGPVDYTCTATGY | 15 |
| 109 | EFSATATYDASPGLI | 15 |
| 110 | TATYDASPGLIGS | 13 |

Moreover, the following sequences form part of the disclosure of the application:
Sequence Total Quantity: 128

### Sequences:

Sequence Number (ID): 1
Length: 508
Molecule Type: AA
Features Location/Qualifiers:
   - REGION, 1..508
      > note, full-length
   - source, 1..508
      > mol_type, protein
      > organism, Bovine Herpes Virus Type I
Residues: 
MGPLGRAWLI AAIFAWALLS ARRGLAEEAE ASPSPPPSPS
PTETESSAGT TGATPPTPNS 60
PDATPEDSTP GATTPVGTPE PPSVSEHDPP VTNSTPPPAP
PEDGRPGGAG NASRDGRPSG 120
GGRPRPPRPS KAPPKERKWM LCEREAVAAS YAEPLYVHCG
VADNATGGAR LELWFQRVGR 180
FRSTRGDDEA VRNPFPRAPP VLLFVAQNGS IAYRSAELGD
NYIFPSPADP RNLPLTVRSL 240
TAATEGVYTW RRDMGTKSQR KVVTVTTHRA PAVSVEPQPA
LEGAGYAAVC RAAEYYPPRS 300
TRLHWFRNGY PVEARHARDV FTVDDSGLFS RTSVLTLEDA
TPTAHPPNLR CDVSWFQSAN 360
MERRFYAAGT PAVYRPPELR VYFEGGEAVC EARCVPEGRV
SLRWTVRDGI APSRTEQTGV 420
CAERPGLVNL RGVRLLSTTD GPVDYTCTAT GYPAPLPEFS
ATATYDASPG LIGSPVLVSV 480
VAVACGLGAV GLLLVAASCL RRKARARL
508
Sequence Number (ID): 2
Length: 508
Molecule Type: AA
Features Location/Qualifiers:
   - REGION, 1..508
      > note, full-length with variants
   - VARIANT, 40
      > note, Xaa stands for Ser or Cys
   - VARIANT, 53
      > note, Xaa stands for Ala or Thr
   - VARIANT, 76
      > note, Xaa stands for Val or Gly
   - VARIANT, 128
      > note, Xaa stands for Arg or Gln
   - VARIANT, 136
      > note, Xaa stands for Glu or Gly
   - VARIANT, 138
      > note, Xaa stands for Lys or Glu
   - VARIANT, 143
      > note, Xaa stands for Glu or Lys
   - VARIANT, 168
      > note, Xaa stands for Gly or Asn
   - VARIANT, 205
      > note, Xaa stands for Val or Ala
   - VARIANT, 206
      > note, Xaa stands for Val or Ala
   - VARIANT, 220
      > note, Xaa stands for Asp or Gly
   - VARIANT, 238
      > note, Xaa stands for Arg or Cys
   - VARIANT, 256
      > note, Xaa stands for Thr or Ala
   - VARIANT, 278
      > note, Xaa stands for Gln or Arg
   - VARIANT, 282
      > note, Xaa stands for Glu or Gln
   - VARIANT, 294
      > note, Xaa stands for Glu or Lys
   - VARIANT, 304
      > note, Xaa stands for His or Arg
   - VARIANT, 356
      > note, Xaa stands for Phe or Tyr
   - VARIANT, 397
      > note, Xaa stands for Glu or Gly
   - VARIANT, 416
      > note, Xaa stands for Glu or Gly
   - VARIANT, 420
      > note, Xaa stands for Val or Ala
   - VARIANT, 472
      > note, Xaa stands for Ile or Val
   - source, 1..508
      > mol_type, protein
      > organism, Bovine Herpes Virus Type I
Residues: 
MGPLGRAWLI AAIFAWALLS ARRGLAEEAE ASPSPPPSPX
PTETESSAGT TGXTPPTPNS 60
PDATPEDSTP GATTPXGTPE PPSVSEHDPP VTNSTPPPAP
PEDGRPGGAG NASRDGRPSG 120
GGRPRPPXPS KAPPKXRXWM LCXREAVAAS YAEPLYVHCG
VADNATGXAR LELWFQRVGR 180
FRSTRGDDEA VRNPFPRAPP VLLFXXQNGS IAYRSAELGX
NYIFPSPADP RNLPLTVXSL 240
TAATEGVYTW RRDMGXKSQR KVVTVTTHRA PAVSVEPXPA
LXGAGYAAVC RAAXYYPPRS 300
TRLXWFRNGY PVEARHARDV FTVDDSGLFS RTSVLTLEDA
TPTAHPPNLR CDVSWXQSAN 360
MERRFYAAGT PAVYRPPELR VYFEGGEAVC EARCVPXGRV
SLRWTVRDGI APSRTXQTGX 420
 
CAERPGLVNL RGVRLLSTTD GPVDYTCTAT GYPAPLPEFS
ATATYDASPG LXGSPVLVSV 480
VAVACGLGAV GLLLVAASCL RRKARARL
508
Sequence Number (ID): 3
Length: 449
Molecule Type: AA
Features Location/Qualifiers:
   - REGION, 1..449
      > note, soluble domain
   - source, 1..449
      > mol_type, protein
      > organism, Bovine Herpes Virus Type I
Residues: 
AEEAEASPSP PPSPSPTETE SSAGTTGATP PTPNSPDATP
EDSTPGATTP VGTPEPPSVS 60
EHDPPVTNST PPPAPPEDGR PGGAGNASRD GRPSGGGRPR
PPRPSKAPPK ERKWMLCERE 120
AVAASYAEPL YVHCGVADNA TGGARLELWF QRVGRFRSTR
GDDEAVRNPF PRAPPVLLFV 180
AQNGSIAYRS AELGDNYIFP SPADPRNLPL TVRSLTAATE
GVYTWRRDMG TKSQRKVVTV 240
TTHRAPAVSV EPQPALEGAG YAAVCRAAEY YPPRSTRLHW
FRNGYPVEAR HARDVFTVDD 300
SGLFSRTSVL TLEDATPTAH PPNLRCDVSW FQSANMERRF
YAAGTPAVYR PPELRVYFEG 360
GEAVCEARCV PEGRVSLRWT VRDGIAPSRT EQTGVCAERP
GLVNLRGVRL LSTTDGPVDY 420
TCTATGYPAP LPEFSATATY DASPGLIGS
449
Sequence Number (ID): 4
Length: 449
Molecule Type: AA
Features Location/Qualifiers:
   - REGION, 1..449
      > note, soluble domain with variants
   - VARIANT, 15
      > note, Xaa stands for Ser or Cys
   - VARIANT, 28
      > note, Xaa stands for Ala or Thr
   - VARIANT, 51
      > note, Xaa stands for Val or Gly
   - VARIANT, 103
      > note, Xaa stands for Arg or Gln
   - VARIANT, 111
      > note, Xaa stands for Glu or Gly
   - VARIANT, 113
      > note, Xaa stands for Lys or Glu
   - VARIANT, 118
      > note, Xaa stands for Glu or Lys
   - VARIANT, 143
      > note, Xaa stands for Gly or Asn
   - VARIANT, 180
      > note, Xaa stands for Val or Ala
   - VARIANT, 181
      > note, Xaa stands for Val or Ala
   - VARIANT, 195
      > note, Xaa stands for Asp or Gly
   - VARIANT, 213
      > note, Xaa stands for Arg or Cys
   - VARIANT, 231
      > note, Xaa stands for Thr or Ala
   - VARIANT, 253
      > note, Xaa stands for Gln or Arg
   - VARIANT, 257
      > note, Xaa stands for Glu or Gln
   - VARIANT, 269
      > note, Xaa stands for Glu or Lys
   - VARIANT, 279
      > note, Xaa stands for His or Arg
   - VARIANT, 331
      > note, Xaa stands for Phe or Tyr
   - VARIANT, 372
      > note, Xaa stands for Glu or Gly
   - VARIANT, 391
      > note, Xaa stands for Glu or Gly
   - VARIANT, 395
      > note, Xaa stands for Val or Ala
   - VARIANT, 447
      > note, Xaa stands for Ile or Val
   - source, 1..449
      > mol_type, protein
      > organism, Bovine Herpes Virus Type I
Residues: 
AEEAEASPSP PPSPXPTETE SSAGTTGXTP PTPNSPDATP
EDSTPGATTP XGTPEPPSVS 60
EHDPPVTNST PPPAPPEDGR PGGAGNASRD GRPSGGGRPR
PPXPSKAPPK XRXWMLCXRE 120
AVAASYAEPL YVHCGVADNA TGXARLELWF QRVGRFRSTR
GDDEAVRNPF PRAPPVLLFX 180
XQNGSIAYRS AELGXNYIFP SPADPRNLPL TVXSLTAATE
GVYTWRRDMG XKSQRKVVTV 240
TTHRAPAVSV EPXPALXGAG YAAVCRAAXY YPPRSTRLXW
FRNGYPVEAR HARDVFTVDD 300
SGLFSRTSVL TLEDATPTAH PPNLRCDVSW XQSANMERRF
YAAGTPAVYR PPELRVYFEG 360
GEAVCEARCV PXGRVSLRWT VRDGIAPSRT XQTGXCAERP
GLVNLRGVRL LSTTDGPVDY 420
TCTATGYPAP LPEFSATATY DASPGLXGS
449
Sequence Number (ID): 5
Length: 507
Molecule Type: AA
Features Location/Qualifiers:
   - REGION, 1..507
      > note, soluble domain expression construct
   - source, 1..507
      > mol_type, protein
      > organism, Bovine Herpes Virus Type I
Residues: 
METDTLLLWV LLLWVPGSTG DAAQPARRAE EAEASPSPPP
SPSPTETESS AGTTGATPPT 60
PNSPDATPED STPGATTPVG TPEPPSVSEH DPPVTNSTPP
PAPPEDGRPG GAGNASRDGR 120
PSGGGRPRPP RPSKAPPKER KWMLCEREAV AASYAEPLYV
HCGVADNATG GARLELWFQR 180
VGRFRSTRGD DEAVRNPFPR APPVLLFVAQ NGSIAYRSAE
LGDNYIFPSP ADPRNLPLTV 240
RSLTAATEGV YTWRRDMGTK SQRKVVTVTT HRAPAVSVEP
QPALEGAGYA AVCRAAEYYP 300
PRSTRLHWFR NGYPVEARHA RDVFTVDDSG LFSRTSVLTL
EDATPTAHPP NLRCDVSWFQ 360
SANMERRFYA AGTPAVYRPP ELRVYFEGGE AVCEARCVPE
GRVSLRWTVR DGIAPSRTEQ 420
TGVCAERPGL VNLRGVRLLS TTDGPVDYTC TATGYPAPLP
EFSATATYDA SPGLIGSGGS 480
GGSSGLNDIF EAQKIEWHEG SHHHHHH
507
Sequence Number (ID): 6
Length: 15
Molecule Type: AA
Features Location/Qualifiers:
   - source, 1..15
      > mol_type, protein
      > organism, Bovine Herpes Virus Type I
Residues:
   AEEAEASPSP PPSPS 15
Sequence Number (ID): 7
Length: 15
Molecule Type: AA
Features Location/Qualifiers:
   - source, 1..15
      > mol_type, protein
      > organism, Bovine Herpes Virus Type I
Residues:
   EASPSPPPSP SPTET 15
Sequence Number (ID): 8
Length: 15
Molecule Type: AA
Features Location/Qualifiers:
   - source, 1..15
      > mol_type, protein
      > organism, Bovine Herpes Virus Type I
Residues:
   SPPPSPSPTE TESSA 15
Sequence Number (ID): 9
Length: 15
Molecule Type: AA
Features Location/Qualifiers:
   - source, 1..15
      > mol_type, protein
      > organism, Bovine Herpes Virus Type I
Residues:
   SPSPTETESS AGTTG 15
Sequence Number (ID): 10
Length: 15
Molecule Type: AA
Features Location/Qualifiers:
   - source, 1..15
      > mol_type, protein
      > organism, Bovine Herpes Virus Type I
Residues:
   TETESSAGTT GATPP 15
Sequence Number (ID): 11
Length: 15
Molecule Type: AA
Features Location/Qualifiers:
   - source, 1..15
      > mol_type, protein
      > organism, Bovine Herpes Virus Type I
Residues:
   SSAGTTGATP PTPNS 15
Sequence Number (ID): 12
Length: 15
Molecule Type: AA
Features Location/Qualifiers:
   - source, 1..15
      > mol_type, protein
      > organism, Bovine Herpes Virus Type I
Residues:
   TTGATPPTPN SPDAT 15
Sequence Number (ID): 13
Length: 15
Molecule Type: AA
Features Location/Qualifiers:
   - source, 1..15
      > mol_type, protein
      > organism, Bovine Herpes Virus Type I
Residues:
   TPPTPNSPDA TPEDS 15
Sequence Number (ID): 14
Length: 15
Molecule Type: AA
Features Location/Qualifiers:
   - source, 1..15
      > mol_type, protein
      > organism, Bovine Herpes Virus Type I
Residues:
   PNSPDATPED STPGA 15
Sequence Number (ID): 15
Length: 15
Molecule Type: AA
Features Location/Qualifiers:
   - source, 1..15
      > mol_type, protein
      > organism, Bovine Herpes Virus Type I
Residues:
   DATPEDSTPG ATTPV 15
Sequence Number (ID): 16
Length: 15
Molecule Type: AA
Features Location/Qualifiers:
   - source, 1..15
      > mol_type, protein
      > organism, Bovine Herpes Virus Type I
Residues:
   EDSTPGATTP VGTPE 15
Sequence Number (ID): 17
Length: 15
Molecule Type: AA
Features Location/Qualifiers:
   - source, 1..15
      > mol_type, protein
      > organism, Bovine Herpes Virus Type I
Residues:
   PGATTPVGTP EPPSV 15
Sequence Number (ID): 18
Length: 15
Molecule Type: AA
Features Location/Qualifiers:
   - source, 1..15
      > mol_type, protein
      > organism, Bovine Herpes Virus Type I
Residues:
   TPVGTPEPPS VSEHD 15
Sequence Number (ID): 19
Length: 15
Molecule Type: AA
Features Location/Qualifiers:
   - source, 1..15
      > mol_type, protein
      > organism, Bovine Herpes Virus Type I
Residues:
   TPEPPSVSEH DPPVT 15
Sequence Number (ID): 20
Length: 15
Molecule Type: AA
Features Location/Qualifiers:
   - source, 1..15
      > mol_type, protein
      > organism, Bovine Herpes Virus Type I
Residues:
   PSVSEHDPPV TNSTP 15
Sequence Number (ID): 21
Length: 15
Molecule Type: AA
Features Location/Qualifiers:
   - source, 1..15
      > mol_type, protein
      > organism, Bovine Herpes Virus Type I
Residues:
   EHDPPVTNST PPPAP 15
Sequence Number (ID): 22
Length: 15
Molecule Type: AA
Features Location/Qualifiers:
   - source, 1..15
      > mol_type, protein
      > organism, Bovine Herpes Virus Type I
Residues:
   PVTNSTPPPA PPEDG 15
Sequence Number (ID): 23
Length: 15
Molecule Type: AA
Features Location/Qualifiers:
   - source, 1..15
      > mol_type, protein
      > organism, Bovine Herpes Virus Type I
Residues:
   STPPPAPPED GRPGG 15
Sequence Number (ID): 24
Length: 15
Molecule Type: AA
Features Location/Qualifiers:
   - source, 1..15
      > mol_type, protein
      > organism, Bovine Herpes Virus Type I
Residues:
   PAPPEDGRPG GAGNA 15
Sequence Number (ID): 25
Length: 15
Molecule Type: AA
Features Location/Qualifiers:
   - source, 1..15
      > mol_type, protein
      > organism, Bovine Herpes Virus Type I
Residues:
   EDGRPGGAGN ASRDG 15
Sequence Number (ID): 26
Length: 15
Molecule Type: AA
Features Location/Qualifiers:
   - source, 1..15
      > mol_type, protein
      > organism, Bovine Herpes Virus Type I
Residues:
   PGGAGNASRD GRPSG 15
Sequence Number (ID): 27
Length: 15
Molecule Type: AA
Features Location/Qualifiers:
   - source, 1..15
      > mol_type, protein
      > organism, Bovine Herpes Virus Type I
Residues:
   GNASRDGRPS GGGRP 15
Sequence Number (ID): 28
Length: 15
Molecule Type: AA
Features Location/Qualifiers:
   - source, 1..15
      > mol_type, protein
      > organism, Bovine Herpes Virus Type I
Residues:
   RDGRPSGGGR PRPPR 15
Sequence Number (ID): 29
Length: 15
Molecule Type: AA
Features Location/Qualifiers:
   - source, 1..15
      > mol_type, protein
      > organism, Bovine Herpes Virus Type I
Residues:
   PSGGGRPRPP RPSKA 15
Sequence Number (ID): 30
Length: 15
Molecule Type: AA
Features Location/Qualifiers:
   - source, 1..15
      > mol_type, protein
      > organism, Bovine Herpes Virus Type I
Residues:
   GRPRPPRPSK APPKE 15
Sequence Number (ID): 31
Length: 15
Molecule Type: AA
Features Location/Qualifiers:
   - source, 1..15
      > mol_type, protein
      > organism, Bovine Herpes Virus Type I
Residues:
   PPRPSKAPPK ERKWM 15
Sequence Number (ID): 32
Length: 15
Molecule Type: AA
Features Location/Qualifiers:
   - source, 1..15
      > mol_type, protein
      > organism, Bovine Herpes Virus Type I
Residues:
   SKAPPKERKW MLCER 15
Sequence Number (ID): 33
Length: 15
Molecule Type: AA
Features Location/Qualifiers:
   - source, 1..15
      > mol_type, protein
      > organism, Bovine Herpes Virus Type I
Residues:
   PKERKWMLCE REAVA 15
Sequence Number (ID): 34
Length: 15
Molecule Type: AA
Features Location/Qualifiers:
   - source, 1..15
      > mol_type, protein
      > organism, Bovine Herpes Virus Type I
Residues:
   KWMLCEREAV AASYA 15
Sequence Number (ID): 35
Length: 15
Molecule Type: AA
Features Location/Qualifiers:
   - source, 1..15
      > mol_type, protein
      > organism, Bovine Herpes Virus Type I
Residues:
   CEREAVAASY AEPLY 15
Sequence Number (ID): 36
Length: 15
Molecule Type: AA
Features Location/Qualifiers:
   - source, 1..15
      > mol_type, protein
      > organism, Bovine Herpes Virus Type I
Residues:
   AVAASYAEPL YVHCG 15
Sequence Number (ID): 37
Length: 15
Molecule Type: AA
Features Location/Qualifiers:
   - source, 1..15
      > mol_type, protein
      > organism, Bovine Herpes Virus Type I
Residues:
   SYAEPLYVHC GVADN 15
Sequence Number (ID): 38
Length: 15
Molecule Type: AA
Features Location/Qualifiers:
   - source, 1..15
      > mol_type, protein
      > organism, Bovine Herpes Virus Type I
Residues:
   PLYVHCGVAD NATGG 15
Sequence Number (ID): 39
Length: 15
Molecule Type: AA
Features Location/Qualifiers:
   - source, 1..15
      > mol_type, protein
      > organism, Bovine Herpes Virus Type I
Residues:
   HCGVADNATG GARLE 15
Sequence Number (ID): 40
Length: 15
Molecule Type: AA
Features Location/Qualifiers:
   - source, 1..15
      > mol_type, protein
      > organism, Bovine Herpes Virus Type I
Residues:
   ADNATGGARL ELWFQ 15
Sequence Number (ID): 41
Length: 15
Molecule Type: AA
Features Location/Qualifiers:
   - source, 1..15
      > mol_type, protein
      > organism, Bovine Herpes Virus Type I
Residues:
   TGGARLELWF QRVGR 15
Sequence Number (ID): 42
Length: 15
Molecule Type: AA
Features Location/Qualifiers:
   - source, 1..15
      > mol_type, protein
      > organism, Bovine Herpes Virus Type I
Residues:
   RLELWFQRVG RFRST 15
Sequence Number (ID): 43
Length: 15
Molecule Type: AA
Features Location/Qualifiers:
   - source, 1..15
      > mol_type, protein
      > organism, Bovine Herpes Virus Type I
Residues:
   WFQRVGRFRS TRGDD 15
Sequence Number (ID): 44
Length: 15
Molecule Type: AA
Features Location/Qualifiers:
   - source, 1..15
      > mol_type, protein
      > organism, Bovine Herpes Virus Type I
Residues:
   VGRFRSTRGD DEAVR 15
Sequence Number (ID): 45
Length: 15
Molecule Type: AA
Features Location/Qualifiers:
   - source, 1..15
      > mol_type, protein
      > organism, Bovine Herpes Virus Type I
Residues:
   RSTRGDDEAV RNPFP 15
Sequence Number (ID): 46
Length: 15
Molecule Type: AA
Features Location/Qualifiers:
   - source, 1..15
      > mol_type, protein
      > organism, Bovine Herpes Virus Type I
Residues:
   GDDEAVRNPF PRAPP 15
Sequence Number (ID): 47
Length: 15
Molecule Type: AA
Features Location/Qualifiers:
   - source, 1..15
      > mol_type, protein
      > organism, Bovine Herpes Virus Type I
Residues:
   AVRNPFPRAP PVLLF 15
Sequence Number (ID): 48
Length: 15
Molecule Type: AA
Features Location/Qualifiers:
   - source, 1..15
      > mol_type, protein
      > organism, Bovine Herpes Virus Type I
Residues:
   PFPRAPPVLL FVAQN 15
Sequence Number (ID): 49
Length: 15
Molecule Type: AA
Features Location/Qualifiers:
   - source, 1..15
      > mol_type, protein
      > organism, Bovine Herpes Virus Type I
Residues:
   APPVLLFVAQ NGSIA 15
Sequence Number (ID): 50
Length: 15
Molecule Type: AA
Features Location/Qualifiers:
   - source, 1..15
      > mol_type, protein
      > organism, Bovine Herpes Virus Type I
Residues:
   LLFVAQNGSI AYRSA 15
Sequence Number (ID): 51
Length: 15
Molecule Type: AA
Features Location/Qualifiers:
   - source, 1..15
      > mol_type, protein
      > organism, Bovine Herpes Virus Type I
Residues:
   AQNGSIAYRS AELGD 15
Sequence Number (ID): 52
Length: 15
Molecule Type: AA
Features Location/Qualifiers:
   - source, 1..15
      > mol_type, protein
      > organism, Bovine Herpes Virus Type I
Residues:
   SIAYRSAELG DNYIF 15
Sequence Number (ID): 53
Length: 15
Molecule Type: AA
Features Location/Qualifiers:
   - source, 1..15
      > mol_type, protein
      > organism, Bovine Herpes Virus Type I
Residues:
   RSAELGDNYI FPSPA 15
Sequence Number (ID): 54
Length: 15
Molecule Type: AA
Features Location/Qualifiers:
   - source, 1..15
      > mol_type, protein
      > organism, Bovine Herpes Virus Type I
Residues:
   LGDNYIFPSP ADPRN 15
Sequence Number (ID): 55
Length: 15
Molecule Type: AA
Features Location/Qualifiers:
   - source, 1..15
      > mol_type, protein
      > organism, Bovine Herpes Virus Type I
Residues:
   YIFPSPADPR NLPLT 15
Sequence Number (ID): 56
Length: 15
Molecule Type: AA
Features Location/Qualifiers:
   - source, 1..15
      > mol_type, protein
      > organism, Bovine Herpes Virus Type I
Residues:
   SPADPRNLPL TVRSL 15
Sequence Number (ID): 57
Length: 15
Molecule Type: AA
Features Location/Qualifiers:
   - source, 1..15
      > mol_type, protein
      > organism, Bovine Herpes Virus Type I
Residues:
   PRNLPLTVRS LTAAT 15
Sequence Number (ID): 58
Length: 15
Molecule Type: AA
Features Location/Qualifiers:
   - source, 1..15
      > mol_type, protein
      > organism, Bovine Herpes Virus Type I
Residues:
   PLTVRSLTAA TEGVY 15
Sequence Number (ID): 59
Length: 15
Molecule Type: AA
Features Location/Qualifiers:
   - source, 1..15
      > mol_type, protein
      > organism, Bovine Herpes Virus Type I
Residues:
   RSLTAATEGV YTWRR 15
Sequence Number (ID): 60
Length: 15
Molecule Type: AA
Features Location/Qualifiers:
   - source, 1..15
      > mol_type, protein
      > organism, Bovine Herpes Virus Type I
Residues:
   AATEGVYTWR RDMGT 15
Sequence Number (ID): 61
Length: 15
Molecule Type: AA
Features Location/Qualifiers:
   - source, 1..15
      > mol_type, protein
      > organism, Bovine Herpes Virus Type I
Residues:
   GVYTWRRDMG TKSQR 15
Sequence Number (ID): 62
Length: 15
Molecule Type: AA
Features Location/Qualifiers:
   - source, 1..15
      > mol_type, protein
      > organism, Bovine Herpes Virus Type I
Residues:
   WRRDMGTKSQ RKVVT 15
Sequence Number (ID): 63
Length: 15
Molecule Type: AA
Features Location/Qualifiers:
   - source, 1..15
      > mol_type, protein
      > organism, Bovine Herpes Virus Type I
Residues:
   MGTKSQRKVV TVTTH 15
Sequence Number (ID): 64
Length: 15
Molecule Type: AA
Features Location/Qualifiers:
   - source, 1..15
      > mol_type, protein
      > organism, Bovine Herpes Virus Type I
Residues:
   SQRKVVTVTT HRAPA 15
Sequence Number (ID): 65
Length: 15
Molecule Type: AA
Features Location/Qualifiers:
   - source, 1..15
      > mol_type, protein
      > organism, Bovine Herpes Virus Type I
Residues:
   VVTVTTHRAP AVSVE 15
Sequence Number (ID): 66
Length: 15
Molecule Type: AA
Features Location/Qualifiers:
   - source, 1..15
      > mol_type, protein
      > organism, Bovine Herpes Virus Type I
Residues:
   TTHRAPAVSV EPQPA 15
Sequence Number (ID): 67
Length: 15
Molecule Type: AA
Features Location/Qualifiers:
   - source, 1..15
      > mol_type, protein
      > organism, Bovine Herpes Virus Type I
Residues:
   APAVSVEPQP ALEGA 15
Sequence Number (ID): 68
Length: 15
Molecule Type: AA
Features Location/Qualifiers:
   - source, 1..15
      > mol_type, protein
      > organism, Bovine Herpes Virus Type I
Residues:
   SVEPQPALEG AGYAA 15
Sequence Number (ID): 69
Length: 15
Molecule Type: AA
Features Location/Qualifiers:
   - source, 1..15
      > mol_type, protein
      > organism, Bovine Herpes Virus Type I
Residues:
   QPALEGAGYA AVCRA 15
Sequence Number (ID): 70
Length: 15
Molecule Type: AA
Features Location/Qualifiers:
   - source, 1..15
      > mol_type, protein
      > organism, Bovine Herpes Virus Type I
Residues:
   EGAGYAAVCR AAEYY 15
Sequence Number (ID): 71
Length: 15
Molecule Type: AA
Features Location/Qualifiers:
   - source, 1..15
      > mol_type, protein
      > organism, Bovine Herpes Virus Type I
Residues:
   YAAVCRAAEY YPPRS 15
Sequence Number (ID): 72
Length: 15
Molecule Type: AA
Features Location/Qualifiers:
   - source, 1..15
      > mol_type, protein
      > organism, Bovine Herpes Virus Type I
Residues:
   CRAAEYYPPR STRLH 15
Sequence Number (ID): 73
Length: 15
Molecule Type: AA
Features Location/Qualifiers:
   - source, 1..15
      > mol_type, protein
      > organism, Bovine Herpes Virus Type I
Residues:
   EYYPPRSTRL HWFRN 15
Sequence Number (ID): 74
Length: 15
Molecule Type: AA
Features Location/Qualifiers:
   - source, 1..15
      > mol_type, protein
      > organism, Bovine Herpes Virus Type I
Residues:
   PRSTRLHWFR NGYPV 15
Sequence Number (ID): 75
Length: 15
Molecule Type: AA
Features Location/Qualifiers:
   - source, 1..15
      > mol_type, protein
      > organism, Bovine Herpes Virus Type I
Residues:
   RLHWFRNGYP VEARH 15
Sequence Number (ID): 76
Length: 15
Molecule Type: AA
Features Location/Qualifiers:
   - source, 1..15
      > mol_type, protein
      > organism, Bovine Herpes Virus Type I
Residues:
   FRNGYPVEAR HARDV 15
Sequence Number (ID): 77
Length: 15
Molecule Type: AA
Features Location/Qualifiers:
   - source, 1..15
      > mol_type, protein
      > organism, Bovine Herpes Virus Type I
Residues:
   YPVEARHARD VFTVD 15
Sequence Number (ID): 78
Length: 15
Molecule Type: AA
Features Location/Qualifiers:
   - source, 1..15
      > mol_type, protein
      > organism, Bovine Herpes Virus Type I
Residues:
   ARHARDVFTV DDSGL 15
Sequence Number (ID): 79
Length: 15
Molecule Type: AA
Features Location/Qualifiers:
   - source, 1..15
      > mol_type, protein
      > organism, Bovine Herpes Virus Type I
Residues:
   RDVFTVDDSG LFSRT 15
Sequence Number (ID): 80
Length: 15
Molecule Type: AA
Features Location/Qualifiers:
   - source, 1..15
      > mol_type, protein
      > organism, Bovine Herpes Virus Type I
Residues:
   TVDDSGLFSR TSVLT 15
Sequence Number (ID): 81
Length: 15
Molecule Type: AA
Features Location/Qualifiers:
   - source, 1..15
      > mol_type, protein
      > organism, Bovine Herpes Virus Type I
Residues:
   SGLFSRTSVL TLEDA 15
Sequence Number (ID): 82
Length: 15
Molecule Type: AA
Features Location/Qualifiers:
   - source, 1..15
      > mol_type, protein
      > organism, Bovine Herpes Virus Type I
Residues:
   SRTSVLTLED ATPTA 15
Sequence Number (ID): 83
Length: 15
Molecule Type: AA
Features Location/Qualifiers:
   - source, 1..15
      > mol_type, protein
      > organism, Bovine Herpes Virus Type I
Residues:
   VLTLEDATPT AHPPN 15
Sequence Number (ID): 84
Length: 15
Molecule Type: AA
Features Location/Qualifiers:
   - source, 1..15
      > mol_type, protein
      > organism, Bovine Herpes Virus Type I
Residues:
   EDATPTAHPP NLRCD 15
Sequence Number (ID): 85
Length: 15
Molecule Type: AA
Features Location/Qualifiers:
   - source, 1..15
      > mol_type, protein
      > organism, Bovine Herpes Virus Type I
Residues:
   PTAHPPNLRC DVSWF 15
Sequence Number (ID): 86
Length: 15
Molecule Type: AA
Features Location/Qualifiers:
   - source, 1..15
      > mol_type, protein
      > organism, Bovine Herpes Virus Type I
Residues:
   PPNLRCDVSW FQSAN 15
Sequence Number (ID): 87
Length: 15
Molecule Type: AA
Features Location/Qualifiers:
   - source, 1..15
      > mol_type, protein
      > organism, Bovine Herpes Virus Type I
Residues:
   RCDVSWFQSA NMERR 15
Sequence Number (ID): 88
Length: 15
Molecule Type: AA
Features Location/Qualifiers:
   - source, 1..15
      > mol_type, protein
      > organism, Bovine Herpes Virus Type I
Residues:
   SWFQSANMER RFYAA 15
Sequence Number (ID): 89
Length: 15
Molecule Type: AA
Features Location/Qualifiers:
   - source, 1..15
      > mol_type, protein
      > organism, Bovine Herpes Virus Type I
Residues:
   SANMERRFYA AGTPA 15
Sequence Number (ID): 90
Length: 15
Molecule Type: AA
Features Location/Qualifiers:
   - source, 1..15
      > mol_type, protein
      > organism, Bovine Herpes Virus Type I
Residues:
   ERRFYAAGTP AVYRP 15
Sequence Number (ID): 91
Length: 15
Molecule Type: AA
Features Location/Qualifiers:
   - source, 1..15
      > mol_type, protein
      > organism, Bovine Herpes Virus Type I
Residues:
   YAAGTPAVYR PPELR 15
Sequence Number (ID): 92
Length: 15
Molecule Type: AA
Features Location/Qualifiers:
   - source, 1..15
      > mol_type, protein
      > organism, Bovine Herpes Virus Type I
Residues:
   TPAVYRPPEL RVYFE 15
Sequence Number (ID): 93
Length: 15
Molecule Type: AA
Features Location/Qualifiers:
   - source, 1..15
      > mol_type, protein
      > organism, Bovine Herpes Virus Type I
Residues:
   YRPPELRVYF EGGEA 15
Sequence Number (ID): 94
Length: 15
Molecule Type: AA
Features Location/Qualifiers:
   - source, 1..15
      > mol_type, protein
      > organism, Bovine Herpes Virus Type I
Residues:
   ELRVYFEGGE AVCEA 15
Sequence Number (ID): 95
Length: 15
Molecule Type: AA
Features Location/Qualifiers:
   - source, 1..15
      > mol_type, protein
      > organism, Bovine Herpes Virus Type I
Residues:
   YFEGGEAVCE ARCVP 15
Sequence Number (ID): 96
Length: 15
Molecule Type: AA
Features Location/Qualifiers:
   - source, 1..15
      > mol_type, protein
      > organism, Bovine Herpes Virus Type I
Residues:
   GEAVCEARCV PEGRV 15
Sequence Number (ID): 97
Length: 15
Molecule Type: AA
Features Location/Qualifiers:
   - source, 1..15
      > mol_type, protein
      > organism, Bovine Herpes Virus Type I
Residues:
   CEARCVPEGR VSLRW 15
Sequence Number (ID): 98
Length: 15
Molecule Type: AA
Features Location/Qualifiers:
   - source, 1..15
      > mol_type, protein
      > organism, Bovine Herpes Virus Type I
Residues:
   CVPEGRVSLR WTVRD 15
Sequence Number (ID): 99
Length: 15
Molecule Type: AA
Features Location/Qualifiers:
   - source, 1..15
      > mol_type, protein
      > organism, Bovine Herpes Virus Type I
Residues:
   GRVSLRWTVR DGIAP 15
Sequence Number (ID): 100
Length: 15
Molecule Type: AA
Features Location/Qualifiers:
   - source, 1..15
      > mol_type, protein
      > organism, Bovine Herpes Virus Type I
Residues:
   LRWTVRDGIA PSRTE 15
Sequence Number (ID): 101
Length: 15
Molecule Type: AA
Features Location/Qualifiers:
   - source, 1..15
      > mol_type, protein
      > organism, Bovine Herpes Virus Type I
Residues:
   VRDGIAPSRT EQTGV 15
Sequence Number (ID): 102
Length: 15
Molecule Type: AA
Features Location/Qualifiers:
   - source, 1..15
      > mol_type, protein
      > organism, Bovine Herpes Virus Type I
Residues:
   IAPSRTEQTG VCAER 15
Sequence Number (ID): 103
Length: 15
Molecule Type: AA
Features Location/Qualifiers:
   - source, 1..15
      > mol_type, protein
      > organism, Bovine Herpes Virus Type I
Residues:
   RTEQTGVCAE RPGLV 15
Sequence Number (ID): 104
Length: 15
Molecule Type: AA
Features Location/Qualifiers:
   - source, 1..15
      > mol_type, protein
      > organism, Bovine Herpes Virus Type I
Residues:
   TGVCAERPGL VNLRG 15
Sequence Number (ID): 105
Length: 15
Molecule Type: AA
Features Location/Qualifiers:
   - source, 1..15
      > mol_type, protein
      > organism, Bovine Herpes Virus Type I
Residues:
   AERPGLVNLR GVRLL 15
Sequence Number (ID): 106
Length: 15
Molecule Type: AA
Features Location/Qualifiers:
   - source, 1..15
      > mol_type, protein
      > organism, Bovine Herpes Virus Type I
Residues:
   GLVNLRGVRL LSTTD 15
Sequence Number (ID): 107
Length: 15
Molecule Type: AA
Features Location/Qualifiers:
   - source, 1..15
      > mol_type, protein
      > organism, Bovine Herpes Virus Type I
Residues:
   LRGVRLLSTT DGPVD 15
Sequence Number (ID): 108
Length: 15
Molecule Type: AA
Features Location/Qualifiers:
   - source, 1..15
      > mol_type, protein
      > organism, Bovine Herpes Virus Type I
Residues:
   RLLSTTDGPV DYTCT 15
Sequence Number (ID): 109
Length: 15
Molecule Type: AA
Features Location/Qualifiers:
   - source, 1..15
      > mol_type, protein
      > organism, Bovine Herpes Virus Type I
Residues:
   TTDGPVDYTC TATGY 15
Sequence Number (ID): 110
Length: 15
Molecule Type: AA
Features Location/Qualifiers:
   - source, 1..15
      > mol_type, protein
      > organism, Bovine Herpes Virus Type I
Residues:
   PVDYTCTATG YPAPL 15
Sequence Number (ID): 111
Length: 15
Molecule Type: AA
Features Location/Qualifiers:
   - source, 1..15
      > mol_type, protein
      > organism, Bovine Herpes Virus Type I
Residues:
   TCTATGYPAP LPEFS 15
Sequence Number (ID): 112
Length: 15
Molecule Type: AA
Features Location/Qualifiers:
   - source, 1..15
      > mol_type, protein
      > organism, Bovine Herpes Virus Type I
Residues:
   TGYPAPLPEF SATAT 15
Sequence Number (ID): 113
Length: 15
Molecule Type: AA
Features Location/Qualifiers:
   - source, 1..15
      > mol_type, protein
      > organism, Bovine Herpes Virus Type I
Residues:
   APLPEFSATA TYDAS 15
Sequence Number (ID): 114
Length: 15
Molecule Type: AA
Features Location/Qualifiers:
   - source, 1..15
      > mol_type, protein
      > organism, Bovine Herpes Virus Type I
Residues:
   EFSATATYDA SPGLI 15
Sequence Number (ID): 115
Length: 13
Molecule Type: AA
Features Location/Qualifiers:
   - source, 1..13
      > mol_type, protein
      > organism, Bovine Herpes Virus Type I
Residues:
   TATYDASPGL IGS 13
Sequence Number (ID): 116
Length: 11
Molecule Type: AA
Features Location/Qualifiers:
   - source, 1..11
      > mol_type, protein
      > organism, Bovine Herpes Virus Type I
Residues:
   TPPTPNSPDA T 11
Sequence Number (ID): 117
Length: 7
Molecule Type: AA
Features Location/Qualifiers:
   - source, 1..7
      > mol_type, protein
      > organism, Bovine Herpes Virus Type I
Residues:
   PSVSEHD 7
Sequence Number (ID): 118
Length: 7
Molecule Type: AA
Features Location/Qualifiers:
   - source, 1..7
      > mol_type, protein
      > organism, Bovine Herpes Virus Type I
Residues:
   YAAVCRA 7
Sequence Number (ID): 119
Length: 11
Molecule Type: AA
Features Location/Qualifiers:
   - source, 1..11
      > mol_type, protein
      > organism, Bovine Herpes Virus Type I
Residues:
   TTDGPVDYTC T 11
Sequence Number (ID): 120
Length: 11
Molecule Type: AA
Features Location/Qualifiers:
   - source, 1..11
      > mol_type, protein
      > organism, Bovine Herpes Virus Type I
Residues:
   TATYDASPGL I 11
Sequence Number (ID): 121
Length: 15
Molecule Type: AA
Features Location/Qualifiers:
   - source, 1..15
      > mol_type, protein
      > organism, Bovine Herpes Virus Type I
   - VARIANT, 4
      > note, X stands for A or T
Residues:
   TTGXTPPTPN SPDAT 15
Sequence Number (ID): 122
Length: 15
Molecule Type: AA
Features Location/Qualifiers:
   - source, 1..15
      > mol_type, protein
      > organism, Bovine Herpes Virus Type I
   - VARIANT, 3
      > note, X stands for V or G
Residues:
   TPXGTPEPPS VSEHD 15
Sequence Number (ID): 123
Length: 15
Molecule Type: AA
Features Location/Qualifiers:
   - source, 1..15
      > mol_type, protein
      > organism, Bovine Herpes Virus Type I
   - VARIANT, 15
      > note, X stands for R or Q
Residues:
   RDGRPSGGGR PRPPX 15
Sequence Number (ID): 124
Length: 15
Molecule Type: AA
Features Location/Qualifiers:
   - source, 1..15
      > mol_type, protein
      > organism, Bovine Herpes Virus Type I
   - VARIANT, 1
      > note, X stands for Q or R
   - VARIANT, 5
      > note, X stands for E or Q
Residues:
   XPALXGAGYA AVCRA 15
Sequence Number (ID): 125
Length: 15
Molecule Type: AA
Features Location/Qualifiers:
   - source, 1..15
      > mol_type, protein
      > organism, Bovine Herpes Virus Type I
   - VARIANT, 9
      > note, X stands for E or K
Residues:
   YAAVCRAAXY YPPRS 15
Sequence Number (ID): 126
Length: 15
Molecule Type: AA
Features Location/Qualifiers:
   - source, 1..15
      > mol_type, protein
      > organism, Bovine Herpes Virus Type I
   - VARIANT, 15
      > note, X stands for I or V
Residues:
   EFSATATYDA SPGLX 15
Sequence Number (ID): 127
Length: 13
Molecule Type: AA
Features Location/Qualifiers:
   - source, 1..13
      > mol_type, protein
      > organism, Bovine Herpes Virus Type I
   - VARIANT, 11
      > note, X stands for I or V
Residues:
   TATYDASPGL XGS 13
Sequence Number (ID): 128
Length: 11
Molecule Type: AA
Features Location/Qualifiers:
   - source, 1..11
      > mol_type, protein
      > organism, Bovine Herpes Virus Type I
   - VARIANT, 11
      > note, X stands for I or V
Residues:
   TATYDASPGL X 11

## Claims

1. A method of identifying in a sample from a non-human animal infected with BoHV-1 (Bovine Herpes Virus Type 1), wherein the sample is serum, comprising the steps:
i. contacting the sample with a polypeptide selected from any of
| |
|---|
| TPPTPNSPDATPEDS |
| TPVGTPEPPSVSEHD |
| TPEPPSVSEHDPPVT |
| GDDEAVRNPFPRAPP |
| QPALEGAGYAAVCRA |
| YAAVCRAAEYYPPRS |
| EDATPTAHPPNLRCD |
| EFSATATYDASPGLI |
| TATYDASPGLIGS |
| TPVGTPEPPSVSEHDPPVT |
| QPALEGAGYAAVCRAAEYYPPRS |
| EFSATATYDASPGLIS |
| TPEPPSVSEHD |
| YAAVCRA |
| |
| TATYDASPGLI |
| |
| TPXGTPEPPSVSEHD (wherein the X stands for V or G) |
| |
| XPALXGAGYAAVCRA (wherein the X at position 1 stands for Q or R, and the X at position 5 stands for E or Q) |
| |
| YAAVCRAAXYYPPRS (wherein the X stands for E or K) |
| |
| TATYDASPGLX (wherein the X stands for I or V) |
under conditions that a complex of an antibody specific against BoHV-1 in the sample and the polypeptide can form; or a polypeptide having at least 90% sequence identity, preferably at least 95% sequence identity, with the above listed peptides;
ii. detecting the complex;
wherein the detection of the complex is indicative of an infection with BoHV-1.

2. The method of claim 1 wherein the complex is detected by labeling said complex with a detectable agent, preferably a color reactant, or a fluorescent agent.

3. A method according to claim 1 wherein the detectable agent is conjugated to any of the polypeptides.

4. A method according to claim 3, wherein the detectable agent is selected from biotin*SA-polyHRP20, an enzyme, a fluorophore, or a colorimetric agent.

5. A method according to claims 1-4, wherein the polypeptide is immobilized on a solid support.

6. A method according to claims 1 to 5 wherein the method is an ELISA (enzyme linked immunosorbent assay), preferably a multi-step or single-step method, preferably wherein the ELISA is monophasic.

7. A method according to any of claims 1 - 6, wherein the polypeptide is derived from Bovine Herpes Virus Type 1, wherein the polypeptide is produced in prokaryotic or eukaryotic cells, preferably in CHO or Expi293 or HEK cells or/and wherein the polypeptide comprises an antigenic determinant that is recognized by antibodies specific for BoHV-1, or/and wherein the one or more isolated polypeptides are linked to an indicator reagent, a signal sequence, a stop transfer sequence, an amino acid spacer, a transmembrane domain, a protein purification ligand, a heterologous polypeptide, a moiety that enhances an immune response, a moiety that facilitates purification, a moiety that facilitates polypeptide stability.
